# EUROPEAN PATENT APPLICATION

(11) **EP 4 700 046 A1**
(43) Date of publication of application: **25.02.2026**
(21) Application number: 24832469.1
(22) Date of filing: 27.06.2024
(51) Int. Cl.: C07K 16/28, A61P 35/00, G01N 33/574, A61K 39/00

(54) **ANTI-CXCR2 ANTIBODY AND USE THEREOF**

(30) Priority: 28.06.2023 KR 20230083329
(71) Applicant: Scripps Korea Antibody Institute, Gangwon-do 24341 (KR)
(72) Inventor: SONG, Yong Bhum, Chuncheon-si Gangwon-do 24303 (KR); RAHIMIZADEH, Parastou, Chuncheon-si Gangwon-do 24294 (KR); KIM, Seheon, Chuncheon-si Gangwon-do 24323 (KR); PARK, Eun Mee, Chuncheon-si Gangwon-do 24432 (KR); YOON, Byeong Jun, Chuncheon-si Gangwon-do 24458 (KR); JEON, Hyeyoon, Chuncheon-si Gangwon-do 24430 (KR); KONG, Deok-Hoon, Chuncheon-si Gangwon-do 24212 (KR); LIM, Seoyoen, Chuncheon-si Gangwon-do 24396 (KR); PARK, Jeong-Ran, Chuncheon-si Gangwon-do 24216 (KR)
(74) Representative: V.O.
(86) International application number: PCT/KR2024/009014
(87) International publication number: WO 2025/005692

(57) **Abstract**

The present invention relates to an anti-CXCR2 antibody and uses thereof.

The anti-CXCR2 antibody or the antigen-binding fragment thereof of the present invention can specifically bind to cancer cells expressing CXCR2 and exhibits an anti-tumor effect by inhibiting the CXCL8/CXCR2 signaling pathway, which plays an important role in tumor formation and growth, and thus can be usefully utilized for the treatment of patients with CXCR2 overexpressing cancer. Furthermore, the anti-CXCR2 antibody or the antigen-binding fragment thereof of the present invention can enable the diagnosis of CXCR2 overexpressing cancer by specifically binding to CXCR2.

## Description

### BACKGROUND

### 1. FIELD

The present invention relates to anti-CXCR2 antibodies and uses thereof.

### 2. DESCRIPTION OF RELATED ART

Chemokines or chemoattractant cytokines typically comprise a family of secreted inducible molecules of small molecular weight (~8 to 10 KDa) that function as activation factors and chemoattractants for leukocytes and can modulate angiogenesis, wound healing, and tumor formation. Due to the influence of chemokines on immune regulation and regulation of inflammation, most information regarding chemokine function is obtained from immune system research. Roles associated with many other biological systems are gradually being revealed.

Chemokines are typically classified into four subfamilies based on the number of conserved cysteine residues at their amino terminus. Most chemokines belong to two major subfamilies having four cysteine residues. These subfamilies are typically classified according to the presence or absence of amino acids between the two amino terminal cysteine residues, and are thus named CC and CXC chemokines. CXC chemokines are typically limited to higher vertebrates, and are generally further classified according to the presence or absence of a glutamate-lysine-arginine (ELR) motif at the amino terminus adjacent to the first cysteine residue. CXCL1, formerly known as Gro-*a*, is a member of the ELR family of CXC chemokines whose preferred receptor is CXCR2.

Each chemokine receptor generally binds to a single class of chemokines, but may bind to several members of the same class with high affinity. Additionally, one chemokine may bind to several different chemokine receptors. For example, CXCR2 may bind to CXCL1, CXCL2, CXCL3, CXCL5, CXCL6, CXCL7, and CXCL8.

The CXCR2 is a type A GPCR belonging to the chemokine receptor family having a size of ~41 kDa. CXCR2 is the sole high-affinity receptor for all pro-angiogenic chemokines (CXCL1-3, CXCL5-8). CXCL6 and CXCL8 (IL-8) also interact with CXCR1 to induce chemotactic effects. Physiologically, CXCR2 is involved in the mobilization and recruitment of leukocytes (particularly neutrophils) from the bone marrow to inflammatory sites and in the migration of endothelial cells in angiogenesis.

Furthermore, CXCR2 has been reported to be expressed in various cell types related to inflammation and cancer, such as neutrophils, granulocytes, endothelial cells, primary cancer cells, and cancer stem cells. Moreover, the CXCLs/CXCR2 signaling axis has been reported as a signaling system that plays a crucial role in various cancers. CXCR2 is highly expressed in several cancer types and is also a bad biomarker for cancer. Furthermore, many studies report that CXCR2 controls cancer cell proliferation through downstream signal activation or signal exchange with other cell surface receptors. And, CXCR2 expressing cancer cells also migrate according to the CXCL8 concentration gradient. Additionally, *in vitro* and *in vivo* studies report that CXCL8/CXCR2 signaling is also involved in apoptosis or angiogenesis. Accordingly, the inhibition or control of CXCR2 signaling can be highly important in achieving an anti-tumor effect.

In this regard, the present inventors have diligently worked to develop an anti-CXCR2 antibody and, as a result, newly generated a human antibody from an antibody library that specifically binds to the CXCR2 protein and can inhibit the CXCL8 signaling pathway, and completed the present invention by confirming that the antibody effectively inhibits tumor formation in CXCR2 overexpressing cancer cells.

### SUMMARY

One object of the present invention is to provide an anti-CXCR2 antibody or an antigen-binding fragment thereof.

Another object of the present invention is to provide a polynucleotide encoding the anti-CXCR2 antibody or the antigen-binding fragment thereof, an expression vector comprising the polynucleotide, and a transformant into which the expression vector is introduced.

Still another object of the present invention is to provide a pharmaceutical composition for the prevention or treatment of cancer, comprising the anti-CXCR2 antibody or the antigen-binding fragment thereof, a polynucleotide encoding the anti-CXCR2 antibody or the antigen-binding fragment thereof, or an expression vector comprising the polynucleotide.

Still another object of the present invention is to provide a pharmaceutical composition for anti-cancer auxiliary use, comprising the anti-CXCR2 antibody or the antigen-binding fragment thereof, a polynucleotide encoding the anti-CXCR2 antibody or the antigen-binding fragment thereof, or an expression vector comprising the polynucleotide, as an active ingredient.

Still another object of the present invention is to provide a method for treating cancer, comprising the step of administering the anti-CXCR2 antibody or the antigen-binding fragment thereof, a polynucleotide encoding the anti-CXCR2 antibody or the antigen-binding fragment thereof, or an expression vector comprising the polynucleotide, to a subject.

Still another object of the present invention is to provide a method for inhibiting the proliferation of CXCR2 protein expressing tumor cells, comprising the step of contacting a tumor cell expressing CXCR2 protein with the anti-CXCR2 antibody or the antigen-binding fragment thereof.

Still another object of the present invention is to provide a composition for diagnosing CXCR2 overexpressing cancer, comprising the anti-CXCR2 antibody or the antigen-binding fragment thereof, a polynucleotide encoding the anti-CXCR2 antibody or the antigen-binding fragment thereof, or an expression vector comprising the polynucleotide.

Still another object of the present invention is to provide a diagnostic kit for CXCR2 overexpressing cancer, comprising the composition and instructions.

Still another object of the present invention is to provide a method for providing information necessary for the diagnosis of CXCR2 overexpressing cancer, comprising the steps of: (a) contacting a biological sample isolated from a subject suspected of having CXCR2 overexpressing cancer with the anti-CXCR2 antibody or the antigen-binding fragment thereof, a polynucleotide encoding the anti-CXCR2 antibody or the antigen-binding fragment thereof, or an expression vector comprising the polynucleotide; (b) measuring the expression level of the CXCR2 protein from the biological sample; and (c) determining that the subject has CXCR2 overexpressing cancer if the expression level of the CXCR2 protein measured in the step (b) is higher than that of a control group.

The present invention for achieving the above objects relates to an anti-CXCR2 antibody or an antigen-binding fragment thereof, comprising any one heavy chain variable region and light chain variable region selected from 1) and 2) below:
1) a heavy chain variable region comprising a CDR1 region consisting of the amino acid sequence of SEQ ID NO: 7, a CDR2 region consisting of the amino acid sequence of SEQ ID NO: 8, and a CDR3 region consisting of the amino acid sequence of SEQ ID NO: 9, and a light chain variable region comprising a CDR1 region consisting of the amino acid sequence of SEQ ID NO: 10, a CDR2 region consisting of the amino acid sequence of SEQ ID NO: 11, and a CDR3 region consisting of the amino acid sequence of SEQ ID NO: 12; and
2) a heavy chain variable region comprising a CDR1 region consisting of the amino acid sequence of SEQ ID NO: 19, a CDR2 region consisting of the amino acid sequence of SEQ ID NO: 20, and a CDR3 region consisting of the amino acid sequence of SEQ ID NO: 21, and a light chain variable region comprising a CDR1 region consisting of the amino acid sequence of SEQ ID NO: 22, a CDR2 region consisting of the amino acid sequence of SEQ ID NO: 23, and a CDR3 region consisting of the amino acid sequence of SEQ ID NO: 24.

In one embodiment of the present invention, the anti-CXCR2 antibody or the antigen-binding fragment thereof may comprise: 1) a heavy chain variable region consisting of the amino acid sequence of SEQ ID NO: 25 and a light chain variable region consisting of the amino acid sequence of SEQ ID NO: 26; or 2) a heavy chain variable region consisting of the amino acid sequence of SEQ ID NO: 27 and a light chain variable region consisting of the amino acid sequence of SEQ ID NO: 28.

In one embodiment of the present invention, the anti-CXCR2 antibody or the antigen-binding fragment thereof may have the activity of inhibiting the growth or metastasis of cancer cells expressing the CXCR2 protein and inducing apoptosis.

In one embodiment of the present invention, the anti-CXCR2 antibody or the antigen-binding fragment thereof can specifically bind to CXCR2 (C-X-C motif chemokine receptor 2).

In one embodiment of the present invention, the anti-CXCR2 antibody or the antigen-binding fragment thereof may have the activity of blocking the binding of CXCL8 (CXC motif chemokine ligand 8) and CXCR2.

In one embodiment of the present invention, the anti-CXCR2 antibody may be a monoclonal antibody.

In one embodiment of the present invention, the antigen-binding fragment may be any one selected from Fab, Fab', F(ab')2, scFv, Fv, dsFv, diabody, Fd, and Fd'.

Furthermore, the present invention for achieving the other aforementioned object relates to a polynucleotide encoding the anti-CXCR2 antibody or the antigen-binding fragment thereof.

In one embodiment of the present invention, the polynucleotide may consist of any one sequence selected from SEQ ID NO: 30 and SEQ ID NO: 32.

Furthermore, the present invention for achieving the still another aforementioned object relates to an expression vector comprising the polynucleotide.

Furthermore, the present invention for achieving the still another aforementioned object relates to a transformant transformed with the expression vector.

Furthermore, the present invention for achieving the still another aforementioned object relates to a pharmaceutical composition for the prevention or treatment of cancer, comprising the anti-CXCR2 antibody or the antigen-binding fragment thereof, a polynucleotide encoding the anti-CXCR2 antibody or the antigen-binding fragment thereof, or an expression vector comprising the polynucleotide, as an active ingredient.

In one embodiment of the present invention, the cancer may be one or more selected from esophageal cancer, stomach cancer, colon cancer, rectal cancer, oral cancer, pharyngeal cancer, laryngeal cancer, lung cancer, colonic cancer, rectal cancer, melanoma, breast cancer, cervical cancer, endometrial cancer, ovarian cancer, prostate cancer, testicular cancer, bladder cancer, kidney cancer, liver cancer, pancreatic cancer, bone cancer, connective tissue cancer, skin cancer, brain cancer, thyroid cancer, leukemia, Hodgkin's disease, soft tissue sarcoma, lymphoma, and multiple myeloma blood cancer.

Furthermore, the present invention for achieving the still another aforementioned object relates to a pharmaceutical composition for anti-cancer auxiliary use, comprising the anti-CXCR2 antibody or the antigen-binding fragment thereof, a polynucleotide encoding the anti-CXCR2 antibody or the antigen-binding fragment thereof, or an expression vector comprising the polynucleotide, as an active ingredient.

Furthermore, the present invention for achieving the still another aforementioned object relates to a method for treating cancer, comprising the step of administering the anti-CXCR2 antibody or the antigen-binding fragment thereof, a polynucleotide encoding the anti-CXCR2 antibody or the antigen-binding fragment thereof, or an expression vector comprising the polynucleotide to a subject.

Furthermore, the present invention for achieving the still another aforementioned object relates to a method for inhibiting the proliferation of CXCR2 protein expressing tumor cells, comprising the step of contacting a tumor cell expressing CXCR2 protein with the anti-CXCR2 antibody or the antigen-binding fragment thereof.

Furthermore, the present invention for achieving the still another aforementioned object relates to a composition for diagnosing CXCR2 overexpressing cancer, comprising the anti-CXCR2 antibody or the antigen-binding fragment thereof, a polynucleotide encoding the anti-CXCR2 antibody or the antigen-binding fragment thereof, or an expression vector comprising the polynucleotide.

Furthermore, the present invention for achieving the still another aforementioned object relates to a diagnostic kit for CXCR2 overexpressing cancer, comprising the composition and instructions.

Furthermore, the present invention for achieving the still another aforementioned object relates to a method for providing information necessary for the diagnosis of CXCR2 overexpressing cancer, comprising the steps of: (a) contacting a biological sample isolated from a subject suspected of having CXCR2 overexpressing cancer with the anti-CXCR2 antibody or the antigen-binding fragment thereof, a polynucleotide encoding the anti-CXCR2 antibody or the antigen-binding fragment thereof, or an expression vector comprising the polynucleotide; (b) measuring the expression level of the CXCR2 protein from the biological sample; and (c) determining that the subject has CXCR2 overexpressing cancer if the expression level of the CXCR2 protein measured in the step (b) is higher than that of a control group.

The anti-CXCR2 antibody or the antigen-binding fragment thereof of the present invention can specifically bind to cancer cells expressing CXCR2 and exhibits an anti-tumor effect by inhibiting the CXCL8/CXCR2 signaling pathway, which plays an important role in tumor formation and growth, and thus can be usefully utilized for the treatment of patients with CXCR2 overexpressing cancer. Furthermore, the anti-CXCR2 antibody or the antigen-binding fragment thereof can enable the diagnosis of CXCR2 overexpressing cancer by specifically binding to CXCR2.

### BRIEF DESCRIPTION OF DRAWINGS

FIG. 1a is the result of purifying the CXCR2(NTD)-hFc recombinant protein, and FIG. 1b is the result of purifying the SUMO-6XHis-CXCR2(NTD) recombinant protein.
FIG. 2 is the result of producing and purifying full-length IgG antibody candidates (#1, #18, #54, and #56) by subcloning the individual DNA sequences of the variable heavy chain (VH) and variable light chain (VL) into an IgG expression vector, after selecting four unique scFvs (#1, #18, #54, and #56) that bind to hCXCR2(NTD) through a biopanning procedure.
FIG. 3 is a graph showing that the binding strength of the full-length IgG antibody candidates (#18 and #56) and hCXCR2(NTD) increases in a dose-dependent manner.
FIG. 4 is a graph showing that the binding strength of the full-length IgG antibody candidates (#1, #18, #54, and #56) and hCXCR2(NTD) increases in a dose-dependent manner.
FIG. 5 is a graph showing the individual KD values of hCXCR2(NTD) and IgG_18 or IgG_56.
FIG. 6 is the FACS analysis result showing that IgG_18 or IgG_56 according to one embodiment of the present invention strongly binds to HEK293 cells expressing hCXCR2.
FIG. 7 is the result showing the binding affinity of IgG_18 or IgG_56 according to one embodiment of the present invention to HEK293 cells expressing hCXCR1-GFP, hCXCR2-GFP, rhCXCR2-GFP, or mCXCR2-GFP.
FIG. 8 is the result of measuring the intracellular calcium level induced by CXCL8 after treating HEK293 cells expressing CXCR2 with' IgG_18 or IgG_56 according to one embodiment of the present invention, along with CXCL8.
FIG. 9 is the Western blot result showing the change in p-ERK and p-AKT expression levels when CXCL8 is treated to HEK293 cells expressing CXCR2 in the presence of IgG_18 or IgG_56 according to one embodiment of the present invention.
FIG. 10 is the result showing that IgG_18 or IgG_56 according to one embodiment of the present invention strongly binds to various cancer cell lines such as pancreatic cancer (PANC-1, BxPC-3, AsPC-1), ovarian cancer (Hey A8, SKOV-3, OVCAR-3), colon cancer (SNU-C2A), and lung cancer (A427).
FIG. 11 is the result showing that IgG_18 or IgG_56 according to one embodiment of the present invention inhibits the increase of AsPC-1 cell proliferation induced by CXCL8.
FIG. 12 is the result showing that IgG_18 or IgG_56 according to one embodiment of the present invention reduces the increase in the number of AsPC-1 cells passing through the membrane induced by CXCL8, i.e., the migration of AsPC-1 cells. *** means that there is a significant difference at p<0.001 compared to the control group (Mock) (Student's t test).
FIG. 13 is the result showing that IgG_18 or IgG_56 according to one embodiment of the present invention increases the apoptosis of AsPC-1 cells reduced by CXCL8.

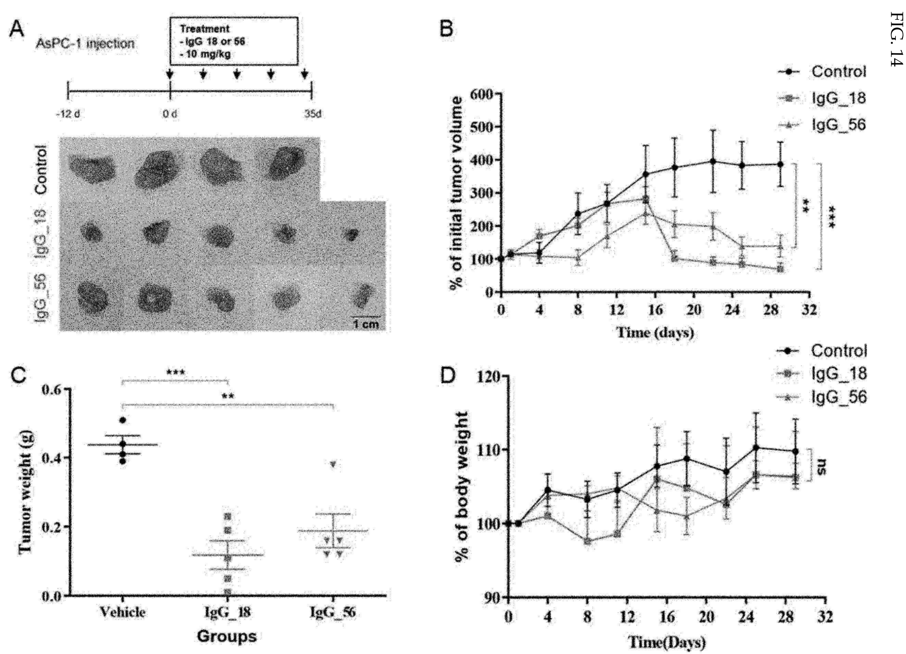

FIGS. 14A-C are the results showing that the tumor volume, size, and weight in mice injected with IgG_18 or IgG_56 according to one embodiment of the present invention are significantly reduced compared to the control group (mice injected with PBS), and FIG. 14D is the result showing that the body weight of mice injected with IgG_18 or IgG_56 according to one embodiment of the present invention shows no significant difference compared to the control group. ** and *** mean that there is a significant difference at *p*<0.01 and *p*<0.001, respectively, compared to the control group (Dunnett's test).

### DETAILED DESCRIPTION

Hereinafter, the present invention will be described in detail.

The present invention provides an anti-CXCR2 antibody or an antigen-binding fragment thereof that specifically binds to CXCR2 (C-X-C motif chemokine receptor 2). The CXCR2 is characterized by being overexpressed in one or more cancers selected from CXCR2 overexpressing cancer, preferably esophageal cancer, stomach cancer, colon cancer, rectal cancer, oral cancer, pharyngeal cancer, laryngeal cancer, lung cancer, colonic cancer, rectal cancer, melanoma, breast cancer, cervical cancer, endometrial cancer, ovarian cancer, prostate cancer, testicular cancer, bladder cancer, kidney cancer, liver cancer, pancreatic cancer, bone cancer, connective tissue cancer, skin cancer, brain cancer, thyroid cancer, leukemia, Hodgkin's disease, soft tissue sarcoma, lymphoma, and multiple myeloma blood cancer, more preferably one or more cancers selected from pancreatic cancer, ovarian cancer, colon cancer, colonic cancer, rectal cancer, lung cancer, melanoma, and prostate cancer, and even more preferably one or more cancers selected from pancreatic cancer, ovarian cancer, colon cancer, and lung cancer.

The CXCR2 plays a role in increasing cancer cell growth by modulating metastasis and angiogenesis of cancer cells. In particular, the inhibition and deficiency of CXCR2 expression in cancer cells not only suppresses angiogenesis but also reduces the growth and metastasis of cancer cells and induces apoptosis. The CXCR2 is known to have high expression in pancreatic cancer, ovarian cancer, colon cancer, colonic cancer, rectal cancer, lung cancer, melanoma, and prostate cancer, and its expression increases upon inflammation.

The present inventors have identified an antibody that specifically recognizes CXCR2, and clarified the amino acid sequence of its heavy chain variable region, the amino acid sequence of its light chain variable region, and the coding nucleotide sequence.

Furthermore, as a preferred embodiment, the present invention provides an anti-CXCR2 antibody or an antigen-binding fragment thereof that specifically binds to CXCR2, thereby specifically binding to cancer cells expressing CXCR2, and exhibiting an anti-tumor effect by inhibiting the CXCL8/CXCR2 signaling pathway which plays an important role in tumor formation and growth, and thus can be usefully utilized for the treatment of patients with CXCR2 overexpressing cancer. Furthermore, it can enable accurate diagnosis of CXCR2 overexpressing cancer cells and/or tissues by specifically detecting CXCR2.

In the present specification, the term "antibody" refers to a protein molecule that functions as a receptor that specifically recognizes an antigen, including an immunoglobulin molecule that is immunologically reactive with a specific antigen, and includes polyclonal antibodies, monoclonal antibodies, complete antibody forms, as well as antigen-binding fragments (antibody fragments) of the antibody molecule. Furthermore, the term includes chimeric antibodies, humanized antibodies, human antibodies, and bivalent or multi-specific antibodies (e.g., bispecific antibodies, trispecific antibodies, etc.), diabodies, triabodies, and tetrabodies.

The "complete antibody (full-length antibody)" is a structure having two full-length light chains and two full-length heavy chains, wherein each light chain is linked to a heavy chain by a disulfide bond. The complete antibody includes IgA, IgD, IgE, IgM, and IgG, and IgG includes subtypes, IgG1, IgG2, IgG3, and IgG4.

The term "antigen-binding fragment of an antibody" herein means a fragment that retains the antigen-antibody binding function within a complete antibody molecule, and includes Fab, Fab', F(ab')2, scFv, Fv, dsFv, diabody, Fd, and Fd', etc. The Fab has a structure comprising the variable regions of the light chain and the heavy chain, the constant region of the light chain, and the first constant region of the heavy chain (CH1 domain), and has one antigen binding site. Fab' differs from Fab in that it has a hinge region comprising one or more cysteine residues at the C-terminus of the heavy chain CH1 domain. The F(ab')2 antibody is produced when the cysteine residues in the hinge region of Fab' form a disulfide bond. Fv (variable fragment) refers to the smallest antibody fragment having only the heavy chain variable region and the light chain variable region. A double-chain Fv (dsFv) has the heavy chain variable region and the light chain variable region linked by a disulfide bond, and a single-chain Fv (scFv) is typically linked by a covalent bond between the heavy chain variable region and the light chain variable region through a peptide linker or directly linked at the C-terminus, thus forming a structure like a dimer, similar to a double-chain Fv. A diabody is a complex of two or more polypeptide chains or proteins, wherein each comprises at least one VL and VH domain or a fragment thereof, and both domains are included within a single polypeptide chain. In some embodiments, the diabody includes molecules comprising an Fc or hinge-Fc domain. The polypeptide chains of such a complex may be the same or different, i.e., the diabody may be a mono-multimer or a hetero-multimer.

Such antigen-binding fragments can be obtained using proteolytic enzymes (e.g., Fab can be obtained by restricting cleavage of a complete antibody with papain, and F(ab')2 fragments can be obtained by cleavage with pepsin), and are preferably produced through genetic recombination technology.

In the present specification, the term "heavy chain" means both a full-length heavy chain comprising the variable region domain VH and three constant region domains CH1, CH2, and CH3, which include an amino acid sequence having a sufficient variable region sequence to confer specificity to an antigen, and a fragment thereof. Furthermore, the term "light chain" herein means both a full-length light chain comprising the variable region domain VL and the constant region domain CL, which include an amino acid sequence having a sufficient variable region sequence to confer specificity to an antigen, and a fragment thereof.

In the present invention, the term "monoclonal antibody" refers to an antibody molecule of a single molecular composition obtained from a substantially homogeneous population of antibodies, and such a monoclonal antibody exhibits single binding specificity and affinity for a specific epitope. Typically, an immunoglobulin has a heavy chain and a light chain, and each heavy chain and light chain comprises a constant region and a variable region (the region is also known as a domain). The variable regions of the light chain and the heavy chain include three hypervariable regions called complementarity-determining regions (hereinafter referred to as "CDRs") and four framework regions (FR). The CDRs primarily function to bind to the epitope of the antigen. The CDRs of each chain are typically called CDR1, CDR2, and CDR3 sequentially starting from the N-terminus, and are also identified by the chain on which the specific CDR is located.

In the present specification, the term "CDR (complementarity determining region)" means the amino acid sequence of the hypervariable region of the immunoglobulin heavy chain and light chain (Kabat et al. Sequences of Proteins of Immunological Interest, 4th Ed., U.S. Department of Health and Human Services, National Institutes of Health (1987)). The heavy chain (CDRH1, CDRH2, and CDRH3) and the light chain (CDRL1, CDRL2, and CDRL3) each comprise three CDRs, and these CDRs provide the major contact residues for the antibody to bind to the antigen or epitope.

Meanwhile, the monoclonal antibody may be a chimeric antibody or a humanized antibody whose immunogenicity is reduced for application to the human body, as described above, or may be a human antibody.

In the present invention, the term "chimeric antibody" refers to an antibody in the form of a recombination of the variable region of a heterogeneous antibody (e.g., mouse, chicken, etc., heterogeneous to humans) and the constant region of a human antibody by DNA recombination technology, and since the immune response of the chimeric antibody is greatly improved compared to heterogeneous antibodies (e.g., mouse, chicken, etc., heterogeneous to humans), it can be used clinically.

In the present invention, the term "humanized antibody" means an antibody in the form of transplanting all or part of the CDR sequence of a heterogeneous monoclonal antibody (e.g., mouse, chicken, etc., heterogeneous to humans) into a human antibody, and examples include manufacturing a humanized variable region by recombining the CDRs of a chicken or mouse monoclonal antibody with FRs derived from a human antibody, and manufacturing the antibody by recombining this with the constant region of a preferred human antibody, but is not limited thereto.

In the present invention, the term "human antibody" possesses an amino acid sequence corresponding to the amino acid sequence of an antibody produced by a human or human cells, or derived from a non-human source using human antibody repertoires or other human antibody coding sequences.

In the present invention, the term "anti-CXCR2 antibody" refers to an antibody that can specifically bind to CXCR2. It includes, without limitation, any antibody that binds to the CXCR2 protein and inhibits the biological activity of CXCR2. Furthermore, the form of the antibody may include complete antibodies, monoclonal antibodies, and antigen-binding fragments, as described above, and may be a chimeric antibody, a humanized antibody, or a human antibody, but is not limited thereto. In addition, the antibody of the present invention specifically binds to CXCR2, inhibits the signaling mediated by CXCR2, and thus inhibits biological activity, so it can be usefully used in the prevention or treatment of cancer involving CXCR2, specifically CXCR2 overexpressing cancer, preferably one or more cancers selected from esophageal cancer, stomach cancer, colon cancer, rectal cancer, oral cancer, pharyngeal cancer, laryngeal cancer, lung cancer, colonic cancer, rectal cancer, melanoma, breast cancer, cervical cancer, endometrial cancer, ovarian cancer, prostate cancer, testicular cancer, bladder cancer, kidney cancer, liver cancer, pancreatic cancer, bone cancer, connective tissue cancer, skin cancer, brain cancer, thyroid cancer, leukemia, Hodgkin's disease, soft tissue sarcoma, lymphoma, and multiple myeloma blood cancer, more preferably one or more cancers selected from pancreatic cancer, ovarian cancer, colon cancer, colonic cancer, rectal cancer, lung cancer, melanoma, and prostate cancer, and even more preferably one or more cancers selected from pancreatic cancer, ovarian cancer, colon cancer, and lung cancer. Furthermore, since overexpression of CXCR2 has been reported as a specific phenomenon in CXCR2 overexpressing cancer, the antibody of the present invention, which can specifically bind to CXCR2, has the diagnostic ability with high sensitivity and specificity in the diagnosis of CXCR2 overexpressing cancer, preferably one or more cancers selected from esophageal cancer, stomach cancer, colon cancer, rectal cancer, oral cancer, pharyngeal cancer, laryngeal cancer, lung cancer, colonic cancer, rectal cancer, melanoma, breast cancer, cervical cancer, endometrial cancer, ovarian cancer, prostate cancer, testicular cancer, bladder cancer, kidney cancer, liver cancer, pancreatic cancer, bone cancer, connective tissue cancer, skin cancer, brain cancer, thyroid cancer, leukemia, Hodgkin's disease, soft tissue sarcoma, lymphoma, and multiple myeloma blood cancer, more preferably one or more cancers selected from pancreatic cancer, ovarian cancer, colon cancer, colonic cancer, rectal cancer, lung cancer, melanoma, and prostate cancer, and even more preferably one or more cancers selected from pancreatic cancer, ovarian cancer, colon cancer, and lung cancer, and thus can be usefully used in the diagnosis of the cancer. In one embodiment of the present invention, the antibody or antigen-binding fragment thereof that specifically binds to CXCR2 was produced using CXCR2 as an antigen protein.

The antibody or an antigen-binding fragment thereof that specifically binds to CXCR2 may comprise any one heavy chain variable region and light chain variable region selected from 1) and 2) below, but is not limited thereto:
1) a heavy chain variable region comprising a CDR1 region consisting of the amino acid sequence of SEQ ID NO: 7, a CDR2 region consisting of the amino acid sequence of SEQ ID NO: 8, and a CDR3 region consisting of the amino acid sequence of SEQ ID NO: 9, and a light chain variable region comprising a CDR1 region consisting of the amino acid sequence of SEQ ID NO: 10, a CDR2 region consisting of the amino acid sequence of SEQ ID NO: 11, and a CDR3 region consisting of the amino acid sequence of SEQ ID NO: 12; and
2) a heavy chain variable region comprising a CDR1 region consisting of the amino acid sequence of SEQ ID NO: 19, a CDR2 region consisting of the amino acid sequence of SEQ ID NO: 20, and a CDR3 region consisting of the amino acid sequence of SEQ ID NO: 21, and a light chain variable region comprising a CDR1 region consisting of the amino acid sequence of SEQ ID NO: 22, a CDR2 region consisting of the amino acid sequence of SEQ ID NO: 23, and a CDR3 region consisting of the amino acid sequence of SEQ ID NO: 24.

In one implementation example of the present invention, the anti-CXCR2 antibody or the antigen-binding fragment thereof may comprise: 1) a heavy chain variable region consisting of the amino acid sequence of SEQ ID NO: 25 and a light chain variable region consisting of the amino acid sequence of SEQ ID NO: 26; or 2) a heavy chain variable region consisting of the amino acid sequence of SEQ ID NO: 27 and a light chain variable region consisting of the amino acid sequence of SEQ ID NO: 28.

All mutants that achieve the desired effect of the present invention through one or more substitutions, deletions, inversions, or translocations in the antibody defined by the above sequence are included within the scope of protection of the present invention.

In one implementation example of the present invention, the anti-CXCR2 antibody or the antigen-binding fragment thereof may have the activity of inducing apoptosis of cancer cells expressing the CXCR2 protein.

In one implementation example of the present invention, the anti-CXCR2 antibody or the antigen-binding fragment thereof can specifically bind to CXCR2 (C-X-C motif chemokine receptor 2).

In one implementation example of the present invention, the anti-CXCR2 antibody or the antigen-binding fragment thereof may have the activity of blocking the binding of CXCL8 (CXC motif chemokine ligand 8) and CXCR2.

In one implementation example of the present invention, the antigen-binding fragment is preferably, but not limited to, Fab, Fab', F(ab')2, scFv, Fv, dsFv, diabody, Fd, Fd', a light chain or heavy chain comprising the CDR region of the present invention, or a Variable domain comprising the CDR region of the present invention.

The anti-CXCR2 antibody or the antigen-binding fragment thereof of the present invention exhibits high apoptotic activity and can inhibit tumor formation in CXCR2 overexpressing cancer, preferably one or more cancers selected from esophageal cancer, stomach cancer, colon cancer, rectal cancer, oral cancer, pharyngeal cancer, laryngeal cancer, lung cancer, colonic cancer, rectal cancer, melanoma, breast cancer, cervical cancer, endometrial cancer, ovarian cancer, prostate cancer, testicular cancer, bladder cancer, kidney cancer, liver cancer, pancreatic cancer, bone cancer, connective tissue cancer, skin cancer, brain cancer, thyroid cancer, leukemia, Hodgkin's disease, soft tissue sarcoma, lymphoma, and multiple myeloma blood cancer, more preferably one or more cancers selected from pancreatic cancer, ovarian cancer, colon cancer, colonic cancer, rectal cancer, lung cancer, melanoma, and prostate cancer, and even more preferably one or more cancers selected from pancreatic cancer, ovarian cancer, colon cancer, and lung cancer.

In another aspect, the present invention provides a polynucleotide encoding the anti-CXCR2 antibody or the antigen-binding fragment thereof.

The polynucleotide may consist of any one sequence selected from SEQ ID NO: 29 to SEQ ID NO: 32, and is preferably, but not limited to, any one sequence selected from SEQ ID NO: 30 and SEQ ID NO: 32.

In yet another aspect, the present invention provides an expression vector comprising the polynucleotide, and a transformant into which the vector is introduced.

The expression vector comprising the polynucleotide encoding the anti-CXCR2 antibody provided by the present invention is not particularly limited thereto, but may be a vector capable of replication and/or expression of the polynucleotide in eukaryotic or prokaryotic cells, including mammalian cells (e.g., human, monkey, rabbit, rat, hamster, mouse cells, etc.), plant cells, yeast cells, insect cells, or bacterial cells (e.g., *E*. *coli,* etc.), and is preferably a vector operably linked to an appropriate promoter so that the nucleotide can be expressed in a host cell, and comprising at least one selectable marker. The example thereof may be a form in which the polynucleotide is introduced into a phage, plasmid, cosmid, mini-chromosome, or virus (e.g., retrovirus, lentivirus, adenovirus, adeno-associated virus, etc.) vector.

The expression vector comprising the polynucleotide encoding the anti-CXCR2 antibody may be an expression vector comprising the polynucleotide encoding the heavy chain or the light chain of the anti-CXCR2 antibody, respectively, or an expression vector comprising both the polynucleotides encoding the heavy chain and the light chain.

The transformant into which the expression vector is introduced, provided by the present invention, is not particularly limited thereto, but may be bacterial cells such as *E*. *coli, Streptomyces, Salmonella typhimurium* transformed with the expression vector; yeast cells; fungal cells such as *Pichia pastoris;* insect cells such as *Drosophila, Spodoptera* Sf9 cells; animal cells such as CHO (Chinese Hamster Ovary cells), SP2/0 (mouse myeloma), human lymphoblastoid, COS, NSO (mouse myeloma), 293T, bow melanoma cells, HT-1080, BHK (baby hamster kidney cells), HEK (human embryonic kidney cells), PERC.6 (human retinal cells); or plant cells.

In the present invention, the term "introduction" refers to a method of delivering a vector comprising the polynucleotide encoding the anti-CXCR2 antibody to a host cell. Such introduction may be performed by various methods known in the art, such as calcium phosphate-DNA co-precipitation method, DEAE-dextran-mediated transfection method, polybrene-mediated transfection method, electroporation method, microinjection method, liposome fusion method, lipofectamine, and protoplast fusion method. Furthermore, transduction means transferring a target substance into a cell using viral particles by means of infection. In addition, the vector can be introduced into the host cell by gene bombardment, etc. In the present invention, introduction may be used interchangeably with transformation.

The host cell of the present invention is preferably an "isolated" host cell.

The host cell may be a host cell derived from a mammal animal other than human.

In yet another aspect, the present invention provides a pharmaceutical composition for the prevention or treatment of cancer, comprising the anti-CXCR2 antibody or the antigen-binding fragment thereof, a polynucleotide encoding the anti-CXCR2 antibody or the antigen-binding fragment thereof, or an expression vector comprising the polynucleotide as an active ingredient.

Since the "anti-CXCR2 antibody or an antigen-binding fragment thereof" of the present invention has already been described above, the description thereof is omitted to avoid excessive redundancy.

In the present invention, the cancer may be one or more selected from esophageal cancer, stomach cancer, colon cancer, rectal cancer, oral cancer, pharyngeal cancer,
laryngeal cancer, lung cancer, colonic cancer, rectal cancer, melanoma, breast cancer, cervical cancer, endometrial cancer, ovarian cancer, prostate cancer, testicular cancer, bladder cancer, kidney cancer, liver cancer, pancreatic cancer, bone cancer, connective tissue cancer, skin cancer, brain cancer, thyroid cancer, leukemia, Hodgkin's disease, soft tissue sarcoma, lymphoma, and multiple myeloma blood cancer. Preferably, the cancer may be one or more selected from pancreatic cancer, ovarian cancer, colon cancer, colonic cancer, rectal cancer, lung cancer, melanoma, and prostate cancer. More preferably, the cancer may be one or more selected from pancreatic cancer, ovarian cancer, colon cancer, and lung cancer. Even more preferably, the cancer may be pancreatic cancer.

In the present invention, the term "prevention" may mean all acts of suppressing or delaying the onset of cancer by administering the composition. Furthermore, the term "treatment" in the present invention may mean all acts of improving or favorably changing the symptoms of cancer by administering the composition.

The pharmaceutical composition may further comprise a pharmaceutically acceptable carrier.

In the present invention, the term "pharmaceutically acceptable carrier" refers to a carrier or diluent that does not stimulate an organism and does not inhibit the biological activity and characteristics of the administered compound. Acceptable pharmaceutical carriers in compositions formulated as liquid solutions include saline, sterile water, Ringer's solution, buffered saline, albumin injection solution, dextrose solution, maltodextrin solution, glycerol, ethanol, and one or more of these ingredients may be mixed and used, and, if necessary, other conventional additives such as antioxidants, buffers, and bacteriostatic agents may be added. Additionally, diluents, dispersants, surfactants, binders, and lubricants may be added to formulate injectable formulations, such as aqueous solutions, suspensions, emulsions, pills, capsules, granules, or tablets.

The pharmaceutical composition may be in various forms for oral or parenteral administration. When formulated, it is prepared using commonly used fillers, extenders, binders, humectants, disintegrants, diluents or excipients such as surfactants. Solid preparations for oral administration include tablets, pills, powders, granules, capsules, etc., and these solid preparations are prepared by mixing one or more compounds with at least one excipient, such as starch, calcium carbonate, sucrose or lactose, gelatin, etc. Furthermore, lubricants such as magnesium stearate and talc are used in addition to simple excipients. Liquid preparations for oral administration include suspensions, internal solutions, emulsions, syrups, etc., and commonly used simple diluents such as water and liquid paraffin, as well as various excipients such as humectants, sweeteners, flavorings, and preservatives, may be included. Preparations for parenteral administration include sterile aqueous solutions, non-aqueous solvents, suspensions, emulsions, lyophilized preparations, and suppositories. Non-aqueous solvents and suspending agents that may be used include propylene glycol, polyethylene glycol, vegetable oils such as olive oil, and injectable esters such as ethyl oleate. Bases for suppositories include witepsol, macrogol, tween 61, cocoa butter, laurin butter, glycerogelatin, etc.

The pharmaceutical composition may have any one formulation selected from the group consisting of tablets, pills, powders, granules, capsules, suspensions, internal solutions, emulsions, syrups, sterile aqueous solutions, non-aqueous solvents, suspensions, emulsions, lyophilized preparations, and suppositories.

The composition of the present invention is administered in a pharmaceutically effective amount.

In the present invention, the term "pharmaceutically effective amount" refers to an amount sufficient to treat a disease with a reasonable benefit/risk ratio applicable to medical treatment, and the effective dose level can be determined according to factors such as the type and severity of the subject, age, gender, type of cancer, activity of the drug, sensitivity to the drug, administration time, administration route and excretion rate, treatment period, drugs used concurrently, and other factors well known in the medical field. The composition of the present invention may be administered as an individual therapeutic agent or in combination with other therapeutic agents, and may be administered sequentially or simultaneously with conventional therapeutic agents. And it can be administered singly or multiply. Considering all the above factors, it is important to administer the amount that achieves the maximum effect with the minimum amount without side effects, and this can be easily determined by those skilled in the art.

In one embodiment of the present invention, it was confirmed that the anti-CXCR2 antibody of the present invention not only specifically binds to CXCR2, but also inhibits the growth or metastasis of cancer cells expressing CXCR2, induces apoptosis, and inhibits tumor formation and growth by inhibiting the CXCL8/ CXCR2 signaling pathway, thereby indicating that the pharmaceutical composition comprising the antibody of the present invention can be usefully used in the prevention or treatment of CXCR2 overexpressing cancer.

In yet another aspect, the present invention provides a pharmaceutical composition for anti-cancer auxiliary use, comprising the anti-CXCR2 antibody or the antigen-binding fragment thereof, a polynucleotide encoding the anti-CXCR2 antibody or the antigen-binding fragment thereof, or an expression vector comprising the polynucleotide, as an active ingredient.

Since the "anti-CXCR2 antibody or an antigen-binding fragment thereof" and "cancer" of the present invention have already been described above, the description thereof is omitted to avoid excessive redundancy.

In the present invention, the term "anti-cancer auxiliary use" means use as an adjuvant for anti-cancer drugs, which is combined with anti-cancer drugs to maximize the effect of the anti-cancer drug by enhancing sensitivity to the anti-cancer drug.

In the present invention, the term "enhancement of sensitivity to anti-cancer drugs" means maximizing the anti-tumor effect by inhibiting anti-cancer drug resistance, where cancer cells acquire non-specific resistance regardless of the structure of the anti-cancer drug.

The anti-cancer drugs that can be combined with the pharmaceutical composition for anti-cancer auxiliary use of the present invention may be, but are not limited to, nitrogen mustard, imatinib, oxaliplatin, rituximab, erlotinib, trastuzumab, gefitinib, bortezomib, sunitinib, carboplatin, sorafenib, bevacizumab, cisplatin, cetuximab, viscum album, asparaginase, tretinoin, hydroxycarbamide, dasatinib, estramustine, gemtuzumab ozogamicin, ibritumomab tiuxetan, heptaplatin, methylaminolevulinate, amsacrine, alemtuzumab, procarbazine, alprostadil, holmium nitrate chitosan, gemcitabine, doxifluridine, pemetrexed, tegafur, capecitabine, gimeracil, oteracil, azacitidine, methotrexate, uracil, cytarabine, fluorouracil, fludarabine, enocitabine, decitabine, mercaptopurine, thioguanine, cladribine, carmofur, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleromycin, daunorubicin, dactinomycin, pirarubicin, aclarubicin, peplomycine, temozolomide, busulfan, ifosfamide, cyclophosphamide, melphalan, altretamine, dacarbazine, thiotepa, nimustine, chlorambucil, mitolactol, lomustine, or carmustine.

In yet another aspect, the present invention provides a method for treating cancer, comprising the step of administering the anti-CXCR2 antibody or the antigen-binding fragment thereof, a polynucleotide encoding the anti-CXCR2 antibody or the antigen-binding fragment thereof, or an expression vector comprising the polynucleotide, to a subject.

Since the "anti-CXCR2 antibody or an antigen-binding fragment thereof" and "cancer" of the present invention have already been described above, the description thereof is omitted to avoid excessive redundancy.

The method for treating cancer may be a method for treating CXCR2 overexpressing cancer, comprising the step of administering a pharmaceutical composition comprising the anti-CXCR2 antibody or the antigen-binding fragment thereof, a polynucleotide encoding the anti-CXCR2 antibody or the antigen-binding fragment thereof, or an expression vector comprising the polynucleotide, and a pharmaceutically acceptable carrier, to a subject in need thereof, and the pharmaceutically acceptable carrier is the same as described above.

The subject includes mammals such as cattle, pigs, sheep, chickens, dogs, and humans, birds, reptiles, amphibians, and fish, and includes, without limitation, a subject whose CXCR2 overexpressing cancer is treated by the administration of the composition of the present invention. The subject may be a mammal other than human.

At this time, the composition may be administered singly or multiply in a pharmaceutically effective amount. At this time, the composition may be administered in the form of a solution, powder, aerosol, capsule, enteric-coated tablet or capsule, or suppository. The administration route includes, but is not limited to, intraperitoneal administration, intravenous administration, intramuscular administration, subcutaneous administration, endothelial administration, oral administration, topical administration, intranasal administration, intrapulmonary administration, and intrarectal administration. However, in the case of oral administration, since peptides are digested, the oral composition must be formulated to coat the active agent or protect it from degradation in the stomach. Furthermore, the pharmaceutical composition may be administered by any device capable of transporting the active substance to the target cell.

Since the pharmaceutical composition of the present invention comprises the antibody that specifically binds to CXCR2 of the present invention, when the pharmaceutical composition comprising the antibody is administered into the human body, it can treat CXCR2 overexpressing cancer by inhibiting the occurrence, proliferation, or metastasis of CXCR2 overexpressing cancer or preventing its progression.

In yet another aspect, the present invention provides a method for inhibiting the proliferation of CXCR2 protein expressing tumor cells, comprising the step of contacting a tumor cell expressing CXCR2 protein with the anti-CXCR2 antibody or the antigen-binding fragment thereof.

Since the "anti-CXCR2 antibody or an antigen-binding fragment thereof" of the present invention has already been described above, the description thereof is omitted to avoid excessive redundancy.

The method may be performed *in-vivo, ex-vivo,* or *in-vitro,* but is preferably performed *ex-vivo* or *in-vitro.*

In yet another aspect, the present invention provides a composition for diagnosing CXCR2 overexpressing cancer, comprising the monoclonal antibody or an antigen-binding fragment thereof, a polynucleotide encoding the monoclonal antibody or an antigen-binding fragment thereof, or an expression vector comprising the polynucleotide.

Since the "anti-CXCR2 antibody or an antigen-binding fragment thereof" of the present invention has already been described above, the description thereof is omitted to avoid excessive redundancy.

The term "overexpression" used to describe CXCR2 overexpressed cancer of the present invention means that when the expression level of CXCR2 is measured through an appropriate expression assay (e.g., Western blot analysis, Peter B. Kaufma et al., Molecular and Cellular Methods in Biology and Medicine, 108-121, CRC press), the expression level is 1.5 times or more, preferably 1.7 times or more, more preferably 2 times or more, and even more preferably 2.3 times or more, compared to the CXCR2 expression level of normal cells, or 1.5 times or more, preferably 1.7 times or more, more preferably 2 times or more, even more preferably 2.5 times or more, and still even more preferably 3 times or more, compared to the CXCR2 expression level of non-malignant cancer cells.

The term "CXCR2 overexpressing cancer" of the present invention may be one or more selected from esophageal cancer, stomach cancer, colon cancer, rectal cancer, oral cancer, pharyngeal cancer, laryngeal cancer, lung cancer, colonic cancer, rectal cancer, melanoma, breast cancer, cervical cancer, endometrial cancer, ovarian cancer, prostate cancer, testicular cancer, bladder cancer, kidney cancer, liver cancer, pancreatic cancer, bone cancer, connective tissue cancer, skin cancer, brain cancer, thyroid cancer, leukemia, Hodgkin's disease, soft tissue sarcoma, lymphoma, and multiple myeloma blood cancer, and preferably one or more cancers selected from pancreatic cancer, ovarian cancer, colon cancer, colonic cancer, rectal cancer, lung cancer, melanoma, and prostate cancer, and more preferably one or more cancers selected from pancreatic cancer, ovarian cancer, colon cancer, and lung cancer.

In the present invention, the term "diagnosis" means confirming the presence, extent (symptoms), and/or characteristics of a pathological condition. As used herein, "diagnosis of CXCR2 overexpressing cancer" means confirming the pathological condition of CXCR2 overexpressing cancer, such as the onset of CXCR2 overexpressing cancer, the extent of the CXCR2 overexpressing cancer site, the location of the CXCR2 overexpressing cancer, and the degree of CXCR2 overexpressing cancer, and it is important to accurately and rapidly distinguish CXCR2 overexpressing cancer cells or CXCR2 overexpressing cancer cells and/or tissue from normal cells or normal tissue for more accurate diagnosis.

As a specific example of the present invention, the present invention provides an antibody that specifically binds to CXCR2, thereby enabling accurate diagnosis of CXCR2 overexpressing cancer cells and/or tissues by specifically detecting CXCR2 at the protein level. Furthermore, a disease related to the expression status or expression level of CXCR2 or a disease mediated by CXCR2, such as CXCR2 overexpressing cancer, can be diagnosed using a diagnostic composition comprising the CXCR2-specific antibody of the present invention.

In yet another aspect, the present invention provides a diagnostic kit for CXCR2 overexpressing cancer, comprising the diagnostic composition for CXCR2 overexpressing cancer.

Since the "anti-CXCR2 antibody or an antigen-binding fragment thereof" and "CXCR2 overexpressing cancer" of the present invention have already been described above, the description thereof is omitted to avoid excessive redundancy.

The diagnostic kit for CXCR2 overexpressing cancer may further comprise a composition, solution, or device having one or more other components suitable for the analysis method. In addition, the diagnostic kit for CXCR2 overexpressing cancer may further comprise instructions.

The present specification provides a means useful for measuring the expression status and/or expression level of CXCR2 by providing an antibody that specifically binds to CXCR2 which is overexpressed in CXCR2 overexpressing cancer, and thereby can be usefully applied to the diagnosis of CXCR2 overexpressing cancer. Furthermore, when the antibody is used in combination with various labeling materials, it can be used for the visualization of CXCR2 overexpressing cancer, and in particular, it can provide more accurate information in confirming the presence of CXCR2 overexpressing cancer, confirming the site of the lesion, and morphological observation of the CXCR2 overexpressing cancer site, and can lower the misdiagnosis rate related to CXCR2 overexpressing cancer and contribute to early diagnosis. Additionally, when a biologically active substance such as a drug is conjugated to the antibody, the biologically active substance can be specifically delivered to colon cancer, so it can be used as a composition for targeted delivery of the biologically active substance to CXCR2 overexpressing cancer.

In yet another aspect, the present invention provides a method for providing information necessary for the diagnosis of CXCR2 overexpressing cancer, comprising the steps of: (a) contacting a biological sample isolated from a subject suspected of having CXCR2 overexpressing cancer with the anti-CXCR2 antibody or the antigen-binding fragment thereof, a polynucleotide encoding the anti-CXCR2 antibody or the antigen-binding fragment thereof, or an expression vector comprising the polynucleotide; (b) measuring the expression level of the CXCR2 protein from the biological sample; and (c) determining that the subject has CXCR2 overexpressing cancer if the expression level of the CXCR2 protein measured in the step (b) is higher than that of a control group.

The anti-CXCR2 antibody, CXCR2 overexpressing cancer, subject, and CXCR2 are the same as described above. The method for providing information necessary for the diagnosis of CXCR2 overexpressing cancer can measure the expression level of the CXCR2 protein through the formation of an antigen-antibody complex by reacting the CXCR2-specific antibody of the present invention with an isolated biological sample from a subject suspected of having CXCR2 overexpressing cancer, thereby providing information for the diagnosis of CXCR2 overexpressing cancer. Since the CXCR2 is overexpressed in CXCR2 overexpressing cancer cells, if the expression level of CXCR2 is higher than that of a control group such as normal cells or tissue, the subject can be determined to have CXCR2 overexpressing cancer.

The provision of information necessary for diagnosis can be utilized as an auxiliary tool for clinicians to diagnose CXCR2 overexpressing cancer.

The provision of information necessary for diagnosis can be performed by a nonclinician, and the clinician can diagnose based on his or her experience by synthesizing the provided information and other test results.

In the present invention, the term "biological sample" includes tissue, cells, whole blood, serum, plasma, tissue autopsy samples (brain, skin, lymph nodes, spinal cord, etc.), saliva, urine, cell culture supernatant, lysed eukaryotic cells, and bacterial expression systems, and is preferably "isolated" *ex vivo.* The presence, expression level of the CXCR2 protein, or whether it is CXCR2 overexpressing cancer can be confirmed by reacting these biological samples, manipulated or unmanipulated, with the antibody of the present invention.

The expression level of the CXCR2 protein can be achieved by measuring the expression level of the CXCR2 protein bound to the anti-CXCR2 antibody or the antigen-binding fragment thereof in the biological sample through the formation of an antigen-antibody complex, but is not limited thereto.

In the present invention, the term "antigen-antibody complex" means a complex of the CXCR2 protein antigen in the sample and the monoclonal antibody according to the present invention that recognizes it, and the formation of such an antigen-antibody complex can be detected by any method selected from the group consisting of colormetric method, electrochemical method, fluorimetric method, luminometry, particle counting method, visual assessment, and scintillation counting method. However, it is not necessarily limited to these, and various applications and adaptations are possible.

In the present invention, various labeling materials can be used to detect the antigen-antibody complex. Specific examples may be selected from the group consisting of enzymes, fluorescent substances, ligands, luminescent substances, microparticles, and radioisotopes, but are not necessarily limited to these.

Enzymes used as detection labels include acetylcholinesterase, alkaline phosphatase, *β*-D-galactosidase, horseradish peroxidase, *β*-lactamase, etc., fluorescent substances include fluorescein, Eu3+, Eu3+ chelate, or cryptate, etc., ligands include biotin derivatives, etc., luminescent substances include acridinium esters, isoluminol derivatives, etc., microparticles include colloidal gold, colored latex, etc., and radioisotopes include 57Co, 3H, 125I, 125I-Bolton Hunter reagent, etc.

Preferably, the antigen-antibody complex can be detected using Enzyme-linked Immunosorbent Assay (ELISA). ELISA includes various ELISA methods such as direct ELISA using a labeled antibody that recognizes an antigen attached to a solid support, indirect ELISA using a labeled secondary antibody that recognizes a capture antibody in a complex of the antigen attached to a solid support and the recognizing antibody, direct sandwich ELISA using another labeled antibody that recognizes the antigen in a complex of the antibody attached to a solid support and the antigen, and indirect sandwich ELISA using another antibody that recognizes the antigen in a complex of the antibody attached to a solid support and the antigen, followed by reacting with a labeled secondary antibody that recognizes this antibody.

The monoclonal antibody may have a detection label, and if it does not have a detection label, it can be confirmed by treating another antibody that can capture these monoclonal antibodies and has a detection label.

In one embodiment of the present invention, it was shown that the antibody of the present invention can be usefully used in the diagnosis of CXCR2 overexpressing cancer by confirming that the anti-CXCR2 antibody of the present invention specifically recognizes CXCR2 using an antigen-antibody reaction.

Hereinafter, the anti-CXCR2 antibody or the antigen-binding fragment thereof according to the present invention will be described in detail with reference to Examples.

### EXAMPLES

### Materials and Methods

### Cell Culture and Transfection

HEK293T and Panc-1 cells were cultured in Dulbecco's modified Eagle's medium (DMEM) supplemented with 10% fetal bovine serum (FBS) and antibiotics, respectively. BxPC-3, AsPC-1, Hey A8, and SNU-C2A cells were cultured in RPMI-1640 medium supplemented with 10% FBS and antibiotics, respectively. HEK293, SKOV-3, and A427 cells were maintained in EMEM, McCoy's 5a, and MEM medium, respectively. OVCAR-3 cells were cultured in RPMI-1640 medium supplemented with 20% FBS. For antigen and antibody production, 293F cells or CHO cells were maintained in FreeStyleTM 293F medium or ExpiCHO Expression medium (Thermo Fisher Scientific).

For stable cell line preparation and maintenance, Geneticin was added to the cell culture medium. Also, plasmids were transfected using Lipofectamine 2000 according to the manufacturer's instructions (Invitrogen).

### DNA Vector Construction

Most expression vectors were constructed using Gateway cloning technology (Invitrogen). Briefly, cDNAs of human CXCR1, human CXCR2, mouse CXCR2, and rhesus monkey CXCR2 were amplified using PCR and then subcloned into the pENTR-D/TOPO vector. Each cDNA in the TOPO vector was recombined into a pcDNA expression vector comprising a C-terminal 3xFLAG-3xHA or GFP tag (Invitrogen). General procedures were performed according to the manufacturer's instructions and previous prior studies [Song YB et al., Trafficking-defective mutant PROKR2 cycles between endoplasmic reticulum and Golgi to attenuate endoplasmic reticulum stress. Proc Natl Acad Sci U S A. 2022 Feb 22;119(8):e2102248119.].

First, to generate the CXCR2(NTD)-hFC protein as an antigen, a DNA sequence encoding the N-terminal domain of human CXCR2 (1 ~ 48 aa) was inserted into the pcDNA3.1 mammalian expression vector. Specifically, the pcDNA3.1-hFC vector was cut with AgeI and XhoI restriction enzymes, and the DNA sequence (CXCR2 1-48 aa, SEQ ID NO: 65) was cloned using the same restriction enzymes to obtain the CXCR2(NTD)-hFC recombinant plasmid.

Subsequently, hCXCR2 (1 ~ 48 aa) was inserted into the pET28a E. coli expression plasmid to produce the 6xHis-SUMO-CXCR2(NTD) protein. Specifically, the pET28a-6xHis-SUMO vector was cut with SacI and XhoI restriction enzymes, and the DNA sequence (CXCR2 1-48 aa, SEQ ID NO: 66) and stop codon TAG were cloned using the same restriction enzymes to obtain the pET28a-His-Sumo-CXCR2(NTD) recombinant plasmid.

Furthermore, to construct the IgG expression vector, cDNA encoding VL and VH having a leader sequence were subcloned into the pdCMV vector between the HindIII-BsiWI and EcoRI-ApaI sites of the plasmid, respectively.

Specifically, the pdCMV-HC expression vector was cut with EcoRI and ApaI restriction enzymes, and the cDNA sequence of each IgG VH (#1; SEQ ID NO: 67, #18; SEQ ID NO: 69, #54; SEQ ID NO: 71, #56; SEQ ID NO: 73) comprising the leader sequence was cloned using the same restriction enzymes to obtain the recombinant plasmid. Also, the pdCMV-LC expression vector was cut with HindIII and BsiWI restriction enzymes, and the cDNA sequence of each IgG VL (#1; SEQ ID NO: 68, #18; SEQ ID NO: 70, #54; SEQ ID NO: 72, #56; SEQ ID NO: 74) comprising the leader sequence was cloned using the same restriction enzymes to obtain the recombinant plasmid.

### Antibodies and Reagents

The antibodies used in this study for flow cytometry analysis, immunoblotting, and immunocytochemistry are shown in Table 1 below. Protein A agarose beads from Amicogen were used for antigen and IgG antibody purification. Ni-NTA agarose was used for antigen purification (QIAGEN). Geneticin was purchased from Gibco, and Calcein AM was purchased from Thermo Fisher Scientific. Recombinant human IL-8/ CXCL8 protein was obtained from R&D system, and SB220050 was purchased from Sigma-Aldrich. All chemicals were dissolved in appropriate reagents according to the manufacturer's instructions.

**Table 1**

| Antibody (source) | Source | Cat# | Company | Use |
|---|---|---|---|---|
| Anti-human IgG conjugated Alexa Flour 647 | Goat | A21445 | TermoFisher | ICC(1:200) |
| Anti-human IgG conjugated Alexa Flour 594 | Goat | A11014 | TermoFisher | ICC(1:200) |
| Peroxidase AffiniPure Goat Anti-Human IgG(H+L) | Goat | 109-035-088 | Jackson Immuno Research | ELISA(1:2500 ) |
| Anti-human FC-HRP | Goat | ab98624 | Abcam | ELISA(1:2500 ) |
| Anti-HA-HRP | Rat | 12013819001 | Roche | ELISA(1:2500 ) |
| Anti-phospho-p44/42 MAPK | Rabbit | 4370 | Cell Signaling Technology | WB(1:2000) |
| Anti-p44/42 MAPK | Rabbit | 4695 | Cell Signaling Technology | WB(1:2000) |
| Anti-Phospho-Akt (Ser473) | Rabbit | 4060 | Cell Signaling Technology | WB(1:2000) |
| Anti-AKT | Rabbit | 4691 | Cell Signaling Technology | WB(1:2000) |
| PE anti-HA. 11 Epitope Tag | mouse | 901517 | BioLegend | ICC(1:1500) |

| | | | | |
|---|---|---|---|---|
| Abbreviation: WB: western blot, ICC: immunocytochemistry | | | | |

### Recombinant Protein and Full IgG Antibody Production

To purify the recombinant protein as an antigen, pcDNA3.1-CXCR2(NTD)-hFC was transfected into FreeStyleTM 293F (Thermo Fisher Scientific) cells, and the recombinant protein was harvested according to the manufacturer's description. For IgG antibody production, both heavy chain (HC) and light chain (LC) expression plasmids were co-transfected into 293F or ExpiCHO cells (Thermo Fisher Scientific). CXCR2(NTD)-hFC and IgG proteins were purified by affinity chromatography using Protein A agarose beads. 6xHis-SUMO-CXCR2(NTD) was produced in an E. coli expression system and purified by affinity chromatography using Ni-NTA agarose (QIAGEN) following size exclusion chromatography using a protein purification system (Cytiva).

### Biopanning Using Phage Display-Based scFv Antibody Library

General procedures for phage display experiments were performed with modification as described in "Phage Display: A Laboratory Manual" [Barbas, C. F., III; Burton, D. R.; Scott, J.K., Silverman, G.J. Eds. (2001) Phage Display: A Laboratory Manual; Cold Spring Harbor Laboratory Press: Cold Spring Harbor, New York, 2001]. Briefly, human IgG or CXCR2(NTD)-hFC for removing hFC binders were coated overnight at 4_{°}C in immunotubes (MaxiSorp, Nunc). Unbound antigen was washed with PBS. After blocking the surface with 3% skim milk in PBST, the scFv phage library was incubated with the coated CXCR2(NTD) on 3% skim milk for 1 hour at room temperature. After washing with PBST to remove unbound phages, captured phages were eluted by incubation with 100 mM triethanolamine for 10 minutes. The eluted phages were used to prepare phages for the next round of panning using amplified ER2738 E. coli strain. Output titers were measured after 3 rounds of panning, and single colonies from the final round were used for the ELISA test. Unique CDR sequences were selected among high-binding clones through sequencing of the scFv DNA and analysis using the IMGT V-quest (www.imgt.org/IMGTindex/V-QUEST.php) analysis program, and through ELISA analysis.

### scFv Expression and Extraction

Single colonies expressing individual scFvs from the binder pool of the third round biopanning were inoculated into 2x YT medium supplemented with carbenicillin (50 ug/ml) and cultured at 37_{°}C for 6 hours. To overexpress the scFv protein, 1 mM of isopropyl β-D-1-thiogalactopyranoside (IPTG) was added to the cell culture medium and cultured overnight at 30_{°}C. After removing the culture medium by centrifugation, the cell pellet was resuspended in 1x TES extraction solution containing 20% w/v sucrose, 50 mM Tris, 1 mM EDTA, pH 8.0. After incubation for 30 minutes at 4_{°}C, 0.2x TES buffer was added to the suspension. After another incubation for 30 minutes at 4_{°}C, the periplasmic protein extract containing the scFv protein was harvested from the supernatant.

### ELISA

The antigen was dissolved in PBS at 1 ug/ml or an appropriate concentration and coated overnight onto high-binding microplates (Corning 96-well Half Area Clear Plate). After washing 3 times with PBS, the surface was coated with 3% skim milk in PBST (0.1% Tween-20) for 1 hour at room temperature. After coating, 30 ul of scFv extract or purified IgG and 3% skim milk solution were incubated with the coated antigen for 1 hour at 37_{°}C. After washing 3 times with PBST, anti-HA-HRP or anti-human FC-HRP was incubated with 3% skim milk solution for 1 hour at 37_{°}C. After an additional washing step, TMB substrate reagent (BD Biosciences) was added and incubated for 5 minutes, then stopped with 1 N H2SO4. Individual optical density (OD) values were measured at 450 nm using a microplate reader.

### Bio-layer Interferometry Kinetic Assay

The Octet R4 protein analysis system (Sartorius) was used for bio-layer interferometry (BLI) kinetic analysis. His-tagged SUMO-CXCR2(NTD) protein (150 nM) was bound to HIS1K biosensors. Candidate antibodies were prepared at 7 concentrations (1.562 nM to 100 nM, half dilution). The antigen-loaded sensor was dipped into the analyte well for 120 seconds and then dissociated in the buffer well for 180 seconds. Unloaded sensors were used as reference sensors and used to subtract the baseline value. Data was generated in Octet Acquisition software and analyzed in Octet Analysis software. Kinetic parameters were evaluated using a 1:1 binding model, and the corresponding KD, Kon, and Koff values were calculated.

### Flow cytometry

Cells were detached from the plate by brief exposure to cell dissociation buffer (Gibco) or 0.25% trypsin/EDTA. After washing with PBS, cells were blocked with 0.1% bovine serum albumin (BSA) in ice-cold PBS for 10 minutes. Cells were treated with an appropriate concentration of anti-CXCR2 candidate substances for 30 minutes at 4_{°}C, and then fluorescently conjugated secondary human IgG was treated for 30 minutes at 4_{°}C. After washing off non-specific binding antibodies, fluorescent data was analyzed using NovoCyte Flow Cytometers (Agilent Technologies, Inc).

### Immunoblotting

Cells were lysed with M-PER Mammalian Protein Extraction Reagent (Thermo Scientific) containing protease inhibitor cocktail (Roche). The extracted protein was reacted with 5x SDS-PAGE sample buffer for 1 hour at room temperature. Protein was separated on an SDS-PAGE gel and then transferred to a nitrocellulose membrane. Bands were detected using a Chemidoc imaging system (Invitrogen).

### Calcium mobilization assay

Intracellular calcium concentration changes were measured according to the manufacturer's instructions (Invitrogen) using the Fluo-4 Direct Calcium Assay Kit. Specifically, HEK293 stable cell line expressing hCXCR2 was cultured in 96-well clear bottom black microplates (Greiner Bio-One). After serum starvation, cells were stained by incubation with Fluo-4 Direct Calcium Buffer containing candidate antibodies for 1 hour at 37_{°}C in a 5% CO2 incubator. Subsequently, after treating with CXCL8, fluorescence intensity was measured using a microplate reader BioTek Synergy H1, Agilent USA.

### Cell migration assay

Cell migration was evaluated using a Boyden chamber (8-µm pore size, Greiner Bio-one). A total of 1.5x105 AsPC-1 cells or 1x105 Hey A8 cells in 150 ul of serum-free medium were added to the upper chamber, and 600 ul of 1% FBS culture medium and 10 nM of CXCL-8 were added to the lower chamber. Subsequently, 20 ug/ml of the candidate antibody was added to the upper chamber. AsPC-1 or Hey A8 cells were incubated for 16 hours or 4 hours, respectively, and then cells were stained with Calcein AM solution for 30 minutes. The number of cells that migrated to the bottom surface of the transwell insert was captured by a fluorescence microscope and counted in 5 randomly selected fields, respectively.

### BrdU cell proliferation assay

Cell proliferation was analyzed using the BrdU Cell Proliferation Assay Kit (Cell Signaling Technology). Specifically, AsPC-1 cells were cultured in RPMI medium containing 10% FBS in a 96-well plate. The next day, cells were starved in serum-free RPMI medium for 3 hours, and then the candidate antibody was added to the medium. After incubation for 1 hour, CXCL8 was treated in the culture medium at a final concentration of 5% FBS. After 72 hours, cells were incubated with BrdU solution for 4 hours, and BrdU incorporation was detected according to the manufacturer's protocol. Individual optical density (OD) values were measured at 450 nm using a microplate reader.

### Annexin-V apoptosis analysis

Apoptotic cells were analyzed according to the manufacturer's instructions (Cell signaling Technology) using the Annexin V-FITC Apoptosis Detection Kit. Specifically, AsPC-1 cells were cultured in RPMI medium containing 10% FBS in a 6-well plate. The next day, cells were starved using serum-free medium for about 1 hour, and then 5% FBS RPMI medium containing CXCL8 or antibody was treated for 72 hours. Cells were harvested and washed twice with PBS, and incubated in Annexin V binding buffer with Annexin V-FITC and PI. Fluorescence of stained cells was analyzed using a flow cytometer.

### Animals and xenograft

Six-week-old BALB/c nude female mice (18-20 g) free of specific pathogens were purchased from a commercial animal breeder (COATECH, Gyeonggi-do, Korea). A total of 20 mice were randomly separated into groups of 5 mice per polycarbonate cage, acclimated for one week in cages controlled at a temperature of 20-24_{°}C and humidity of 45-55%. Mice were given free access to standard rodent chow and water, and cages were maintained on a 12-hour light/dark cycle. After a 7-day acclimation period, a total of 5x106 AsPC-1 pancreatic carcinoma cells in 150 µl of serum-free medium were subcutaneously injected into the right dorsal flank of the nude mice. Twelve days after tumor induction, mice were divided into three groups based on tumor size (80.19±29.06 mm3, range 37.42-127.32 mm3). The control group (4 mice) was intravenously injected with physiological saline, while the antibody treated groups (IgG_18 treated group (5 mice) and IgG_56 treated group (5 mice)) were injected intravenously (i.v.) with 10 mg/kg of each antibody (IgG_18 and IgG_56) once a week for 5 weeks. On the last treatment day, mice were euthanized and xenografts were harvested for further analysis. Tumor size and body weight were measured twice weekly for 5 weeks and on the day of sacrifice. Subcutaneous tumor volume was measured in two dimensions (length and width) using Ultra-Cal VI calipers. Tumor growth inhibition (TGI) as a percentage of vehicle was calculated as the percentage difference between the daily average areas of the treated groups over the same period. All experimental procedures were approved by the Institutional Animal Care and Use Committee of Scripps Korea Antibody Institute (Approval No. SIACUC-22-1-4).

### Test Results

### Screening for Antibodies against Human CXCR2 from a Phage Display Human Library

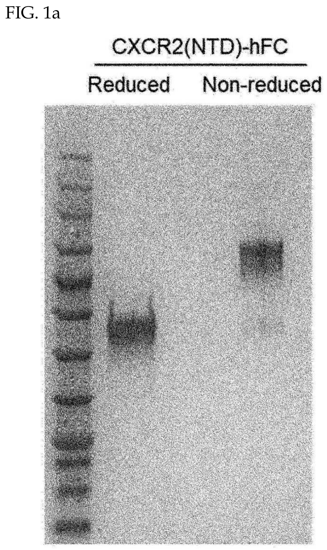

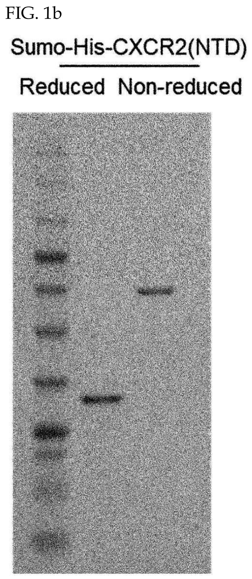

The N-terminal extracellular domain of human CXCR2 is known to be important for ligand binding and specificity [Gayle RB 3rd et al., Importance of the amino terminus of the interleukin-8 receptor in ligand interactions. J Biol Chem. 1993 Apr 5;268(10):7283-9. PMID: 8463264.], [LaRosa GJ et al., Amino terminus of the interleukin-8 receptor is a major determinant of receptor subtype specificity. J Biol Chem. 1992 Dec 15;267(35):25402-6. PMID: 1281158.], [Wu L et al., Discrete steps in binding and signaling of interleukin-8 with its receptor. J Biol Chem. 1996 Dec 6;271(49):31202-9], [Joseph PR et al., Probing the role of CXC motif in chemokine CXCL8 for high affinity binding and activation of CXCR1 and CXCR2 receptors. J Biol Chem. 2010 Sep 17;285(38):29262-9.], [Liu K et al., Structural basis of CXC chemokine receptor 2 activation and signalling. Nature. 2020 Sep;585(7823):135-140.]. Furthermore, crystallographic and nuclear magnetic resonance analysis results also showed the importance of extracellular loop 2 for CXCL8 binding [Katancik JA et al., Mapping of the extracellular binding regions of the human interleukin-8 type B receptor. Biochem Biophys Res Commun. 1997 Mar 27;232(3):663-8.], [Ahuja SK et al., CXC chemokines bind to unique sets of selectivity determinants that can function independently and are broadly distributed on multiple domains of human interleukin-8 receptor B. Determinants of high affinity binding and receptor activation are distinct. J Biol Chem. 1996 Jan 5;271(1):225-32.], [Luo Z et al., Molecular modeling of interleukin-8 receptor beta and analysis of the receptor-ligand interaction. Protein Eng. 1997 Sep;10(9):1039-45.]. In this study, 48 amino acid sequences located at the N-terminal domain (NTD) of the hCXCR2 protein were selected for biopanning. First, a recombinant protein (CXCR2(NTD)-hFC) fused with a human Fc fragment at the C-terminus of CXCR2(NTD) was purified (FIG. 1a). Also, the SUMO-6XHis-CXCR2(NTD) recombinant protein was prepared to remove non-specific binders or proteins interacting with the Fc fragment on the antigen (FIG. 1b). Phage display technology was initially performed to screen for specific binders to the CXCR2(NTD)-Fc protein. Furthermore, the present inventors used a human library comprising human synthetic single-chain variable fragments (scFv) for use in biopanning [Yang HY et al., Construction of a large synthetic human scFv library with six diversified CDRs and high functional diversity. Mol Cells. 2009 Feb 28;27(2):225-35.]. After 3 rounds of biopanning, Enzyme-linked Immunosorbent Assay (ELISA) was performed to initially select the binding antibody group, followed by sequence analysis to select 9 unique scFv sequences.

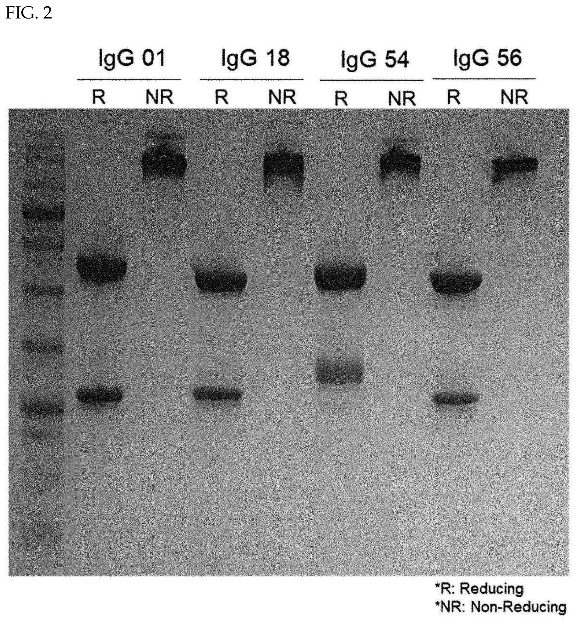

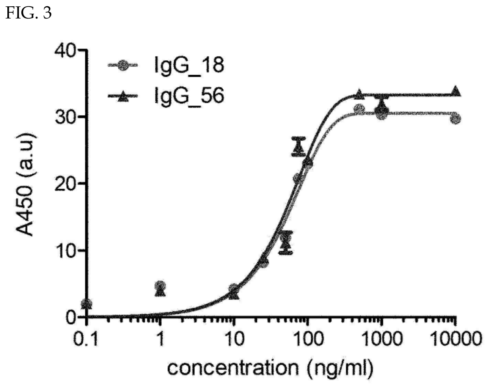

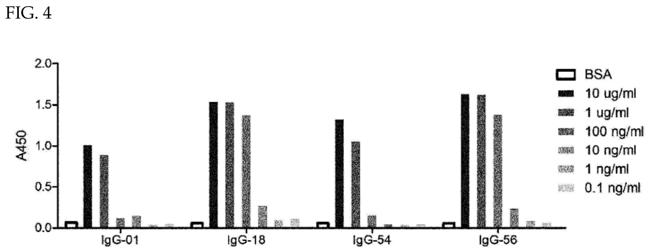

Furthermore, the present inventors additionally confirmed the binding of scFv candidates to a stable HEK293 cell line expressing human CXCR2-GFP. As a result, four unique scFv sequences (#1 (Table 2, SEQ ID NO: 29), #18 (Table 3, SEQ ID NO: 30), #54 (Table 4, SEQ ID NO: 31), and #56 (Table 5, SEQ ID NO: 31)) that bind to hCXCR2(NTD) could be selected using the biopanning procedure. Subsequently, full-length IgG proteins were produced after subcloning the individual DNA sequences of the variable heavy chain (VH) and variable light chain (VL) into an IgG expression vector (FIG. 2). ELISA was performed to confirm the interaction between hCXCR2(NTD) and full-length IgG candidates (#1, #18, #54, and #56), and the strength was shown to increase in a dose-dependent manner (FIG. 3 and FIG. 4).

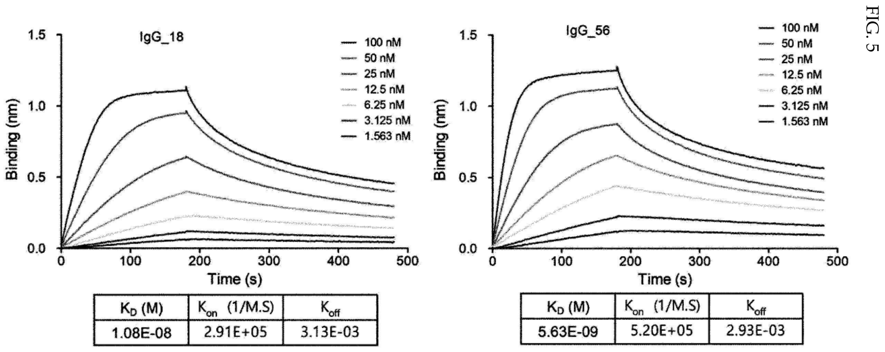

Furthermore, the present inventors accurately analyzed the direct affinity between CXCR2(NTD) and candidate antibodies using BLI (bio-layer interferometry)-based analysis. The individual KD values of hCXCR2(NTD) and IgG_18, IgG_54, and IgG_56 were found to be 8.9 nM, 250 nM, and 5.6 nM, respectively (FIG. 5). However, IgG_1 showed very weak binding compared to the other candidate IgG proteins. Collectively, four human antibodies against hCXCR2(NTD) were screened by biopanning, and IgG_18 and IgG_56 antibodies were specifically selected as antibody candidates targeting hCXCR2.

**Table 2**

| IgG_1 (#1) | | Amino acid sequence | SEQ ID No. |
|---|---|---|---|
| VH | FR1 | EVQLLESGGGLVQPGGSLRLSCAAS | 33 |
| | CDR1 | GFTFSNYS | 1 |
| | FR2 | MSWVRQAPGKGLEWVSW | 34 |
| | CDR2 | IYYNGGST | 2 |
| | FR3 | YYADSVKGRFTISRDNSKNTLYLQMNSLRAEDTAVYYC | 35 |
| | CDR3 | ARGPRICEIRKCYSANAMDV | 3 |
| | FR4 | WGQGTLVTVSS | 36 |
| VL | FR1 | QSVLTQPPSASGTPGQRVTISCSGS | 37 |
| | CDR1 | SSNIGNNA | 4 |
| | FR2 | VSWYQQLPGTAPKLLIY | 38 |
| | CDR2 | ANS | 5 |
| | FR3 | NRPSGVPDRFSGSKSGTSASLAISGLRSEDEADYYC | 39 |
| | CDR3 | GTWDASLSAYV | 6 |
| | FR4 | FGGGTKLTVL | 40 |

**Table 3**

| IgG_18 (#18) | | Amino acid sequence | SEQ ID No. |
|---|---|---|---|
| VH | FR1 | EVQLLESGGGLVQPGGSLRLSCAAS | 41 |
| | CDR1 | GFTFSNYA | 7 |
| | FR2 | MSWVRQAPGKGLEWVSS | 42 |
| | CDR2 | ISPSGSSK | 8 |
| | FR3 | YYADSVKGRFTISRDNSKNTLYLQMNSLRAEDTAVYYC | 43 |
| | CDR3 | AKHNRHFDY | 9 |
| | FR4 | WGQGTLVTVSS | 44 |
| VL | FR1 | QSVLTQPPSASGTPGQRVTISCTGS | 45 |
| | CDR1 | SSNIGSHY | 10 |
| | FR2 | VSWYQQLPGTAPKLLIY | 46 |
| | CDR2 | DNN | 11 |
| | FR3 | HRPSGVPDRFSGSKSGTSASLAISGLRSEDEADYYC | 47 |
| | CDR3 | GSWDDSLNAYV | 12 |
| | FR4 | FGGGTKLTVL | 48 |

**Table 4**

| IgG_54 (#54) | | Amino acid sequence | SEQ ID No. |
|---|---|---|---|
| VH | FR1 | EVQLLESGEGLVQPGGSLRLSCAAS | 49 |
| | CDR1 | GFTFSNYS | 13 |
| | FR2 | MSWVRQAPGKGLEWVSG | 50 |
| | CDR2 | ISSGGGSK | 14 |
| | FR3 | YYADSVKGRFTISRDNSKNTLYLQMNSLRAEDTAVYYC | 51 |
| | CDR3 | ARQATAFDY | 15 |
| | FR4 | WGQGTLVTVSS | 52 |
| VL | FR1 | QSVLTQPPSASGTPGQRVTISCTGS | 53 |
| | CDR1 | SSNIGNNS | 16 |
| | FR2 | VTWYQQLPGTAPKLLIY | 54 |
| | CDR2 | SDS | 17 |
| | FR3 | NRPSGVPDRFSGSKSGTSASLAISGLRSEDEADYYC | 55 |
| | CDR3 | GTWDDSLSAYV | 18 |
| | FR4 | FCGGTKLTVL | 56 |

**Table 5**

| IgG_56 (#56) | | Amino acid sequence | SEQ ID No. |
|---|---|---|---|
| VH | FR1 | EVQLLESGGGLVQPGGSLRLSCAAS | 57 |
| | CDR1 | GFTFSGYP | 19 |
| | FR2 | MSWVRQAPGKGLEWVSS | 58 |
| | CDR2 | ISSSGGNT | 20 |
| | FR3 | YYADSVKGRFTISRDNSKNTLYLQMNSLRAEDTAVYYC | 59 |
| | CDR3 | ARFGIHHPDRALYYANAMDV | 21 |
| | FR4 | WGQGTLVTVSS | 60 |
| VL | FR1 | QSVLTQPPSASGTPGQRVTISCSGS | 61 |
| | CDR1 | SSNIGNND | 22 |
| | FR2 | VNWYQQLPGTAPKLLIY | 62 |
| | CDR2 | ADN | 23 |
| | FR3 | QRPSGVPDRFSGSKSGTSASLAISGLRSEDEADYYC | 63 |
| | CDR3 | ATWDASLSAYV | 24 |
| | FR4 | FGGGTKLTVL | 64 |

### Verification of Antibody Binding to Various Types of CXCR2 Expressing Cells

To confirm whether the IgG_18 or IgG_56 antibody of the present invention has the ability to bind to hCXCR2 expressing cells, antibody binding to stable HEK293 cells expressing hCXCR2-GFP or hCXCR2-3xFLAG-3xHA was monitored using a flow cytometer.

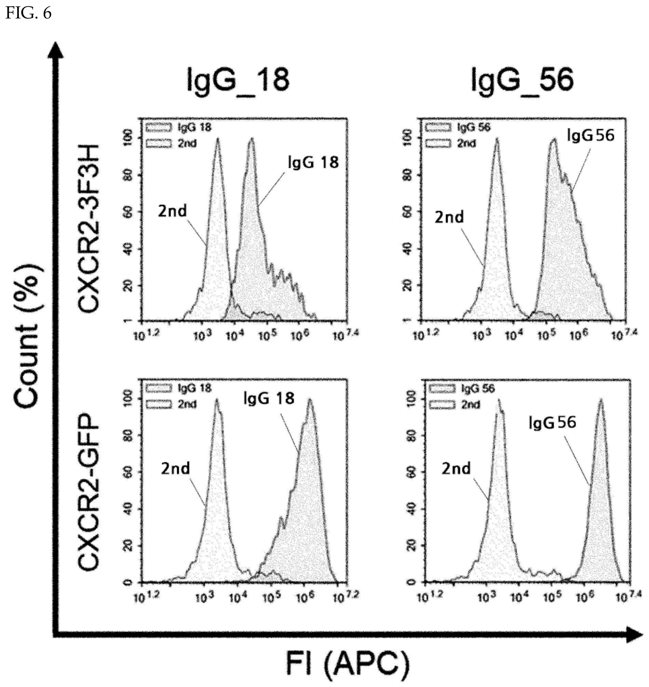

Similar to the affinity measurement results shown in FIGS. 3 to 5, the FACS results showed that both IgG_18 and IgG_56 strongly bound to hCXCR2 expressing HEK293 cells (FIG. 6). The N-terminal domain (1-48 aa) of hCXCR2 showed only 33% identity with the N-terminus of hCXCR1.

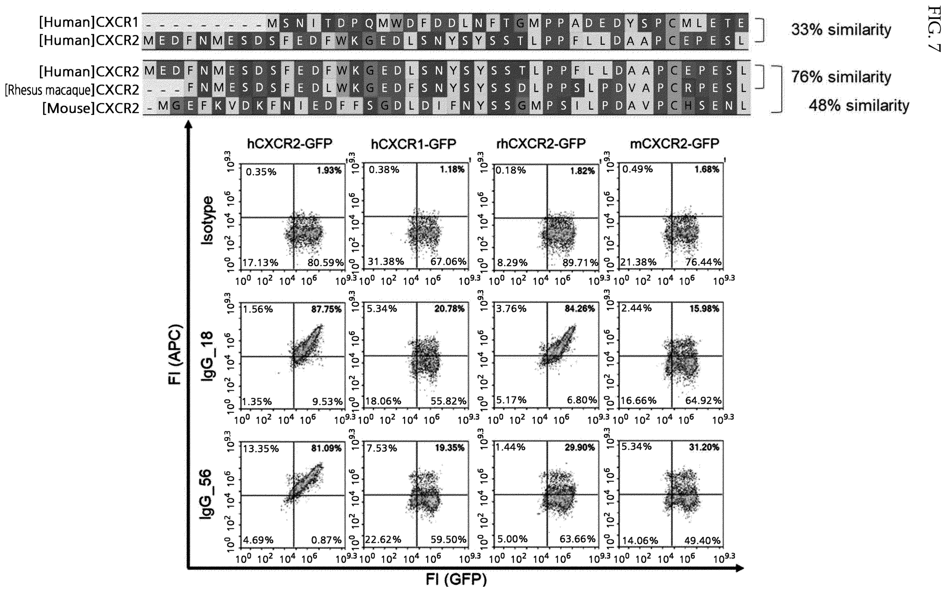

Furthermore, to determine antibody specificity, antibody binding to HEK293 cells transiently expressing hCXCR1 or hCXCR2 was compared. Both IgG18 and IgG56 of the present invention showed strong binding to cells expressing hCXCR2-GFP, but did not show significant binding affinity to cells expressing hCXCR1-GFP (FIG. 7).

Next, candidate antibodies were further analyzed to determine if they had specificity compared to other orthotropic CXCR2 proteins. Antibody binding to cells expressing mouse CXCR2 (mCXCR2-GFP) or rhesus monkey CXCR2 (rhCXCR2-GFP) was monitored. Both IgG_18 and IgG_56 showed no strong binding to mCXCR2-GFP expressing cells (48% identity). Notably, it was revealed that the IgG_18 antibody could bind to rhCXCR2-GFP expressing cells (76% identity), which suggests that it may share similar epitope regions of the hCXCR2 protein (FIG. 7).

From these results, it can be seen that both IgG_18 and IgG_56 of the present invention possess the ability to bind with high affinity to both CXCR2-recombinant protein and CXCR2-expressing cells, and also have specificity against the subtype or heterotype CXCR2 protein of the chemokine receptor.

### CXCR2 Antibody Inhibits CXCL8-Induced Signaling Pathway

Chemokines binding to G protein-coupled chemokine receptors mediate cell mobility through changes in intracellular calcium concentration [Rossi D et al., The biology of chemokines and their receptors. Annu Rev Immunol. 2000;18:217-42.]. It is very well known that CXCR2 can elevate intracellular calcium levels in the presence of an agonist such as CXCL8. Therefore, calcium signaling is a crucial and direct indicator for evaluating CXCR2 activation.

The present inventors expected that if the anti-CXCR2 antibody according to the present invention binds to the CXCR2 receptor and blocks the binding of CXCL8 to the receptor, the calcium elevation induced by CXCL8 treatment would decrease. Accordingly, to evaluate whether IgG_18 and IgG_56 of the present invention both have functional characteristics as antagonist antibodies in the CXCL8/CXCR2 signaling axis, CXCL8-mediated intracellular calcium elevation was first monitored.

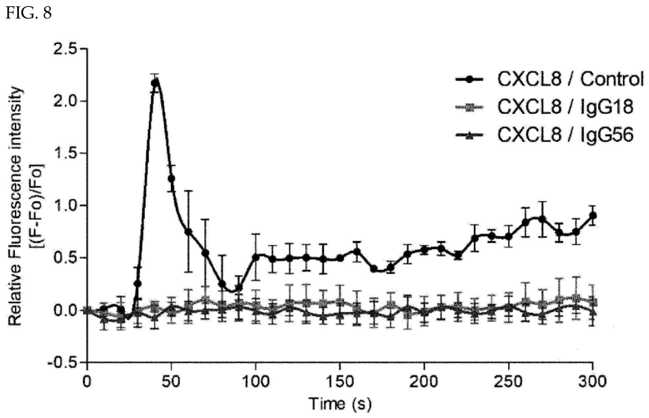

As expected, the intracellular calcium level increased when 10 nM of CXCL8 was treated to stable HEK293 cells expressing CXCR2, while CXCR2 expressing cells in the presence of IgG_18 or IgG_56 did not increase the intracellular calcium level induced by CXCL8 (FIG. 8).

Meanwhile, CXCL8/CXCR2 signaling is related to tumor progression. CXCL8/CXCR2 signaling is known to activate several signaling pathways, including MAPK (mitogen-activated protein kinases) or PI3K (phosphatidylinositol-3 kinase)/Akt pathways, and controls many cellular activities related to cell proliferation, differentiation, motility, or apoptosis [Liu Q et al., The CXCL8-CXCR1/2 pathways in cancer. Cytokine Growth Factor Rev. 2016 Oct;31:61-71.], [Cheng Y et al., Potential roles and targeted therapy of the CXCLs/CXCR2 axis in cancer and inflammatory diseases. Biochim Biophys Acta Rev Cancer. 2019 Apr;1871(2):289-312.].

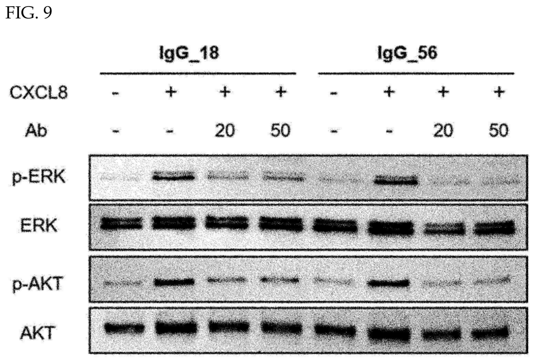

To further verify the CXCL8 binding blocking effect of the CXCR2 antibody, activation monitoring of intracellular signaling mediators was performed. Specifically, phosphorylation of p42/44 protein increased rapidly when CXCL8 was treated to CXCR2 stable HEK293 cells, whereas phosphorylation of p42/44 did not sufficiently increase when CXCL8 was treated to CXCR2 stable HEK293 cells in the presence of IgG_18 or IgG_56 (FIG. 9). Also, consistently with the MAPK pathway, treatment with IgG_18 or IgG_56 could reduce the phosphorylation (activation) of AKT, which was increased by CXCL8 treatment (FIG. 9).

From these results, it can be seen that both IgG_18 and IgG_56 of the present invention function as antagonists to CXCL8/CXCR2 activation.

### CXCR2 Inhibitory Antibody Suppresses Tumor Formation

CXCL8/CXCR2 signaling is important in cancer and inflammatory diseases. Several studies have reported that CXCR2 can play a role in chronic inflammation and an important role in angiogenesis, tumor formation, and cancer metastasis in colon cancer, rectal cancer, melanoma, lung cancer, prostate cancer, ovarian cancer, and pancreatic cancer. [Stadtmann A et al., CXCR2: From Bench to Bedside. Front Immunol. 2012 Aug 24;3:263.], [Gabellini C et al., Functional activity of CXCL8 receptors, CXCR1 and CXCR2, on human malignant melanoma progression. Eur J Cancer. 2009 Sep;45(14):2618-27.], [Ohri CM et al., Chemokine receptor expression in tumour islets and stroma in non-small cell lung cancer. BMC Cancer. 2010 Apr 29;10:172.], [Reiland J et al., CXC-chemokines stimulate invasion and chemotaxis in prostate carcinoma cells through the CXCR2 receptor. Prostate. 1999 Oct 1;41(2):78-88.], [Wang B et al., A growth-related oncogene/CXC chemokine receptor 2 autocrine loop contributes to cellular proliferation in esophageal cancer. Cancer Res. 2006 Mar 15;66(6):3071-7.], [Yang G et al., CXCR2 promotes ovarian cancer growth through dysregulated cell cycle, diminished apoptosis, and enhanced angiogenesis. Clin Cancer Res. 2010 Aug 1;16(15):3875-86.], [Li A et al., CXCR2-Dependent Endothelial Progenitor Cell Mobilization in Pancreatic Cancer Growth. Transl Oncol. 2011 Feb 1;4(1):20-8.]. Overexpression of CXCR2 has been reported to be high not only in immune cells such as neutrophils but also in many primary cancer cells. It has been confirmed through FIG. 6 that the IgG_18 and IgG_56 antibodies according to the present invention specifically bind to hCXCR2 expressing HEK293T cells.

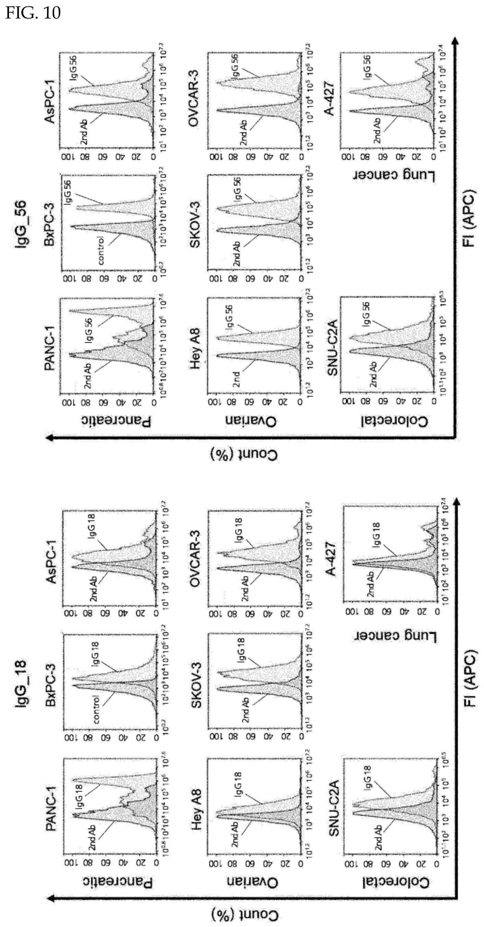

To monitor whether CXCR2 is expressed in various cancer cell lines and whether the CXCR2 antibody can bind to the cancer cell lines, analysis was performed using a flow cytometer after immunostaining. The analysis results confirmed that both IgG18 and IgG56 according to the present invention strongly bind to various cancer cell lines such as pancreatic cancer, ovarian cancer, colon cancer, and lung cancer cell lines (FIG. 10). This supports that CXCR2 is expressed in many cancer cells, and CXCR2 signaling may play an important role in tumor formation.

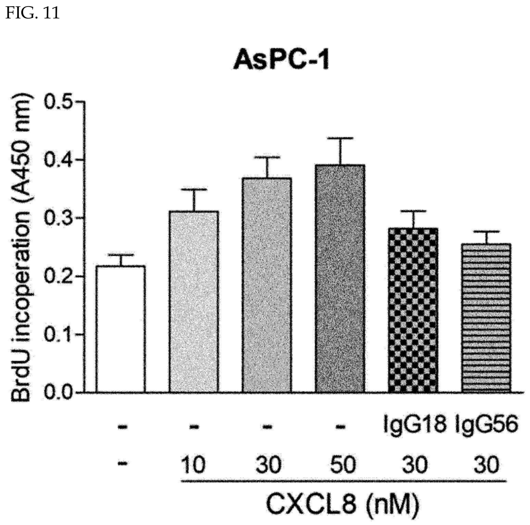

Furthermore, to explore the cancer therapeutic potential of IgG_18 and IgG_56 according to the present invention as inhibitory antibodies, the effect on cell proliferation in pancreatic cancer cell lines was first investigated. As expected, the BrdU cell proliferation assay confirmed that CXCL8 significantly promoted the proliferation of AsPC-1 cells in a dose-dependent manner (FIG. 11). And, this cancer cell proliferation did not sufficiently increase even in the 30 nM CXCL8 treated group in the presence of IgG_18 or IgG_56.

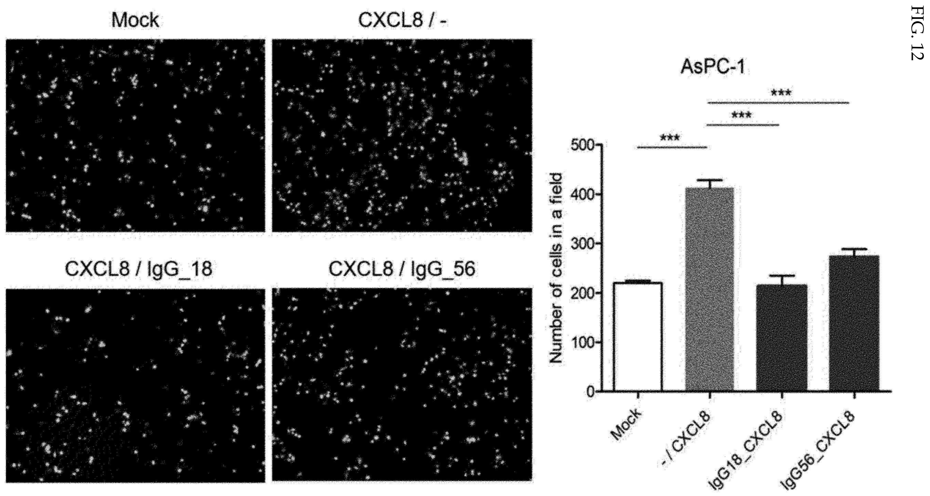

Next, we wanted to further analyze whether the CXCR2 antibody has a functional antagonistic effect on cancer cell migration. As a result, the Transwell cell migration assay confirmed that AsPC-1 cells significantly migrated to the lower chamber towards the high concentration of CXCL8, whereas treatment with IgG_18 or IgG_56 of the present invention significantly reduced the increase in the number of AsPC-1 cells passing through the membrane induced by CXCL8 (FIG. 12).

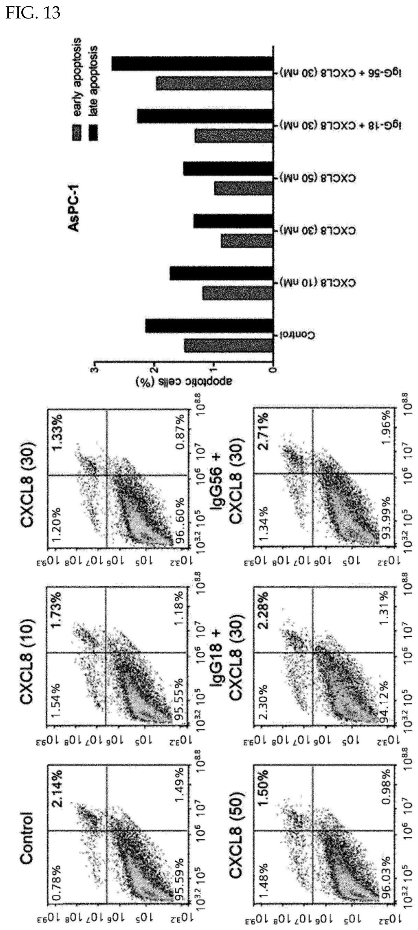

Furthermore, to further evaluate the antagonistic effect of the antibody on cancer cells, the level of apoptosis was monitored. Cancer cell apoptosis was detected using flow cytometry after staining with Annexin V and propidium iodide (PI). As shown in FIG. 13, when AsPC-1 cells were treated with CXCL8, cancer cell apoptosis decreased compared to vehicle-treated cells, whereas when IgG18 or IgG56 of the present invention was treated, the CXCL8-reduced cancer cell apoptosis increased. This result indicates that CXCR2 blockade by the IgG18 or IgG56 antibody of the present invention causes apoptosis in AsPC-1 pancreatic cancer cells.

Collectively, IgG18 and IgG56 according to the present invention can bind to various cancer cells, including pancreatic cancer cells, and can have an anti-tumor effect by inhibiting the CXCL8/CXCR2 signaling pathway in cancer.

### CXCR2 Antibody Attenuates Cancer Growth in Xenograft Mouse Model System

To evaluate the potential therapeutic benefits of the antibody according to the present invention, the anti-tumor effect on tumors *in vivo* was next investigated. A xenograft mouse model in which BALB/c nude mice were subcutaneously injected with AsPC-1 pancreatic carcinoma was used. After allowing the tumors to grow until they reached 80 mm3, mice were administered 10 mg/kg of IgG_18 or IgG_56 (intravenous injection). As shown in FIGS. 14A-C, the tumor volume and size, and the final tumor weight were significantly reduced compared to mice injected with PBS, and notably, mice treated with 10 mg/kg of IgG_18 showed a stronger inhibitory effect on the increase of tumor weight (FIG. 14C). Furthermore, the body weight of the tested mice was not affected by the administration of the candidate antibody, indicating that the antibody had no noticeable side effects in *in vivo* experiments (FIG. 14D).

Collectively, these results show that IgG_18 and IgG_56 exhibit anti-tumor effects by inhibiting CXCR2 signaling in a pancreatic cancer cell xenograft model.

From the above description, those skilled in the art to which the present invention pertains will understand that the present invention can be embodied in other specific forms without changing its technical spirit or essential features. In this regard, the embodiments described above should be understood as being illustrative in all respects and not restrictive. The scope of the present invention should be construed as including all changes or modifications derived from the meaning and scope of the appended claims described below rather than the detailed description above, and their equivalents.

## Claims

1. An anti-CXCR2 antibody or an antigen-binding fragment thereof, comprising any one heavy chain variable region and light chain variable region selected from 1) and 2) below:
1) a heavy chain variable region comprising a CDR1 region consisting of the amino acid sequence of SEQ ID NO: 7, a CDR2 region consisting of the amino acid sequence of SEQ ID NO: 8, and a CDR3 region consisting of the amino acid sequence of SEQ ID NO: 9, and a light chain variable region comprising a CDR1 region consisting of the amino acid sequence of SEQ ID NO: 10, a CDR2 region consisting of the amino acid sequence of SEQ ID NO: 11, and a CDR3 region consisting of the amino acid sequence of SEQ ID NO: 12; and
2) a heavy chain variable region comprising a CDR1 region consisting of the amino acid sequence of SEQ ID NO: 19, a CDR2 region consisting of the amino acid sequence of SEQ ID NO: 20, and a CDR3 region consisting of the amino acid sequence of SEQ ID NO: 21, and a light chain variable region comprising a CDR1 region consisting of the amino acid sequence of SEQ ID NO: 22, a CDR2 region consisting of the amino acid sequence of SEQ ID NO: 23, and a CDR3 region consisting of the amino acid sequence of SEQ ID NO: 24.

2. The anti-CXCR2 antibody or the antigen-binding fragment thereof according to Claim 1, wherein the anti-CXCR2 antibody or the antigen-binding fragment thereof comprises:
1) a heavy chain variable region consisting of the amino acid sequence of SEQ ID NO: 25 and a light chain variable region consisting of the amino acid sequence of SEQ ID NO: 26; or
2) a heavy chain variable region consisting of the amino acid sequence of SEQ ID NO: 27 and a light chain variable region consisting of the amino acid sequence of SEQ ID NO: 28.

3. The anti-CXCR2 antibody or the antigen-binding fragment thereof according to Claim 1, wherein
the anti-CXCR2 antibody or the antigen-binding fragment thereof has the activity of inhibiting the growth or metastasis of cancer cells expressing the CXCR2 protein and inducing apoptosis.

4. The anti-CXCR2 antibody or the antigen-binding fragment thereof according to Claim 1, wherein
the anti-CXCR2 antibody or the antigen-binding fragment thereof blocks the binding of CXCL8 (CXC motif chemokine ligand 8) and CXCR2.

5. The anti-CXCR2 antibody or the antigen-binding fragment thereof according to Claim 1, wherein
the anti-CXCR2 antibody is a monoclonal antibody.

6. The anti-CXCR2 antibody or the antigen-binding fragment thereof according to Claim 1, wherein
the antigen-binding fragment is any one selected from Fab, Fab', F(ab')2, scFv, Fv, dsFv, diabody, Fd, and Fd'.

7. A polynucleotide encoding the anti-CXCR2 antibody or the antigen-binding fragment thereof according to any one of Claims 1 to 6.

8. The polynucleotide according to Claim 7, wherein
the polynucleotide consists of any one sequence selected from SEQ ID NO: 30 and SEQ ID NO: 32.

9. An expression vector comprising the polynucleotide of Claim 7.

10. A transformant transformed with the expression vector of Claim 9.

11. A pharmaceutical composition for the prevention or treatment of cancer, comprising the anti-CXCR2 antibody or the antigen-binding fragment thereof according to any one of Claims 1 to 6, a polynucleotide encoding the anti-CXCR2 antibody or the antigen-binding fragment thereof, or an expression vector comprising the polynucleotide, as an active ingredient.

12. The composition according to Claim 11, wherein
the cancer is one or more selected from esophageal cancer, stomach cancer, colon cancer, rectal cancer, oral cancer, pharyngeal cancer, laryngeal cancer, lung cancer, colonic cancer, rectal cancer, melanoma, breast cancer, cervical cancer, endometrial cancer, ovarian cancer, prostate cancer, testicular cancer, bladder cancer, kidney cancer, liver cancer, pancreatic cancer, bone cancer, connective tissue cancer, skin cancer, brain cancer, thyroid cancer, leukemia, Hodgkin's disease, soft tissue sarcoma, lymphoma, and multiple myeloma blood cancer.

13. A composition for diagnosing CXCR2 overexpressing cancer, comprising the anti-CXCR2 antibody or the antigen-binding fragment thereof according to any one of Claims 1 to 6, a polynucleotide encoding the anti-CXCR2 antibody or the antigen-binding fragment thereof, or an expression vector comprising the polynucleotide.

14. A diagnostic kit for CXCR2 overexpressing cancer, comprising the composition of Claim 13 and instructions.

15. A method for providing information necessary for the diagnosis of CXCR2 overexpressing cancer, comprising the steps of:
(a) contacting a biological sample isolated from a subject suspected of having CXCR2 overexpressing cancer with the anti-CXCR2 antibody or the antigen-binding fragment thereof according to any one of Claims 1 to 6, a polynucleotide encoding the anti-CXCR2 antibody or the antigen-binding fragment thereof, or an expression vector comprising the polynucleotide;
(b) measuring the expression level of the CXCR2 protein from the biological sample; and
(c) determining that the subject has CXCR2 overexpressing cancer if the expression level of the CXCR2 protein measured in the step (b) is higher than that of a control group.

16. A method for treating cancer, comprising the step of administering the anti-CXCR2 antibody or the antigen-binding fragment thereof according to any one of Claims 1 to 6, a polynucleotide encoding the anti-CXCR2 antibody or the antigen-binding fragment thereof, or an expression vector comprising the polynucleotide, to a subject.
